# EUROPEAN PATENT APPLICATION

(11) **EP 4 553 159 A1**
(43) Date of publication of application: **14.05.2025**
(21) Application number: 23835605.9
(22) Date of filing: 06.07.2023
(51) Int. Cl.: C12N 15/54, A23L 33/125, A23L 33/20, C12N 1/15, C12N 1/19, C12N 1/21, C12N 5/10, C12N 9/10, C12N 15/63, C12P 7/02

(54) **STEVIOL GLYCOSIDE BIOSYNTHESIS MUTANT ENZYME AND USE OF SAME**

(30) Priority: 07.07.2022 JP 2022110052
(71) Applicant: Suntory Holdings Limited, Osaka-shi, Osaka 530-8203 (JP)
(72) Inventor: HIRAI Tadayoshi, Soraku-gun, Kyoto 619-0284 (JP); ONO Eiichiro, Soraku-gun, Kyoto 619-0284 (JP); TANAKA Yoshikazu, Soraku-gun, Kyoto 619-0284 (JP); OCHIAI Kentaro, Soraku-gun, Kyoto 619-0284 (JP); MIZOHATA Eiichi, Suita-shi, Osaka 565-0871 (JP)
(74) Representative: Mewburn Ellis LLP
(86) International application number: PCT/JP2023/025199
(87) International publication number: WO 2024/010074

(57) **Abstract**

The present invention provides a protein having an amino acid sequence of SEQ ID NO: 2 and a mutant thereof, a polynucleotide encoding the protein, a transformant including the polynucleotide, and a steviol glycoside using the protein and a method for producing a product containing the same.

## Description

### Technical Field

The present invention relates to a protein having an activity of transferring a sugar to a steviol glycoside, a polynucleotide encoding the protein, a transformant including the polynucleotide, a method for producing a steviol glycoside or a product including the steviol glycoside using the protein or the transformant, and the like.

### Background Art

The leaves of Stevia rebaudiana belonging to the family Asteraceae contain a secondary metabolite called steviol, a kind of diterpenoid, and a steviol glycoside, which is a glycoside thereof, has a sweetness that is about 300 times greater than that of sucrose and thus is used in the food industry as a non-caloric sweetener. Obesity has been internationally developing as a serious social issue, and there is a growing demand for non-caloric sweeteners from the viewpoint of health promotion and medical cost reduction. At present, artificially synthesized amino acid derivatives such as aspartame and acesulfame potassium are used as artificial sweeteners, but naturally occurring non-caloric sweeteners such as steviol glycosides are expected to be safer and easily gain public acceptance.

Steviol glycosides of stevia are synthesized by adding sugars to steviol, which is an aglycone, by various glycosyltransferases. Each steviol glycoside has unique taste characteristics. Among them, rebaudioside D (hereinafter, rebaudioside may be abbreviated as "Reb") and RebM not only have high sweetness but also exhibit good sweetness with less aftertaste of sweetness, bitterness, and the like. However, steviol glycosides having such good taste characteristics are contained only in a trace amount in stevia, and thus attempts have been made to artificially synthesize steviol glycosides with a glycosyltransferase. Some glycosyltransferases involved in steviol glycoside biosynthesis have been isolated, and mutants thereof have also been developed (Patent Literatures 1 to 3 and Non-Patent Literatures 1 and 2).

### Citation List

### Patent Literature

Patent Literature 1: WO 2011/153378
Patent Literature 2: WO 2013/137487
Patent Literature 3: WO 2018/124141

### Non-Patent Literature

Non-Patent Literature 1: Brandle and Telmer (2007) Phytochemistry 68, 1855-1863
Non-Patent Literature 2: Richman et al (2005) Plant J. 41, 56-67

### Summary of Invention

### Technical Problem

There is a need for a glycosyltransferase that can synthesize a desired steviol glycoside in a high yield.

### Solution to Problem

The present invention provides a protein or the like having high activity to add hexose at position 2 in a monomeric glucose residue at position 13 or position 19 of a steviol glycoside.

In one embodiment, the present invention provides the following.
[1] A protein selected from:
   (a) a protein including the amino acid sequence of SEQ ID NO: 2;
   (b) a protein including an amino acid sequence wherein 1 to 48 amino acid residues other than the amino acid residue at position 155 are deleted, substituted, inserted, and/or added in the amino acid sequence of SEQ ID NO: 2 and having an activity to add hexose at position 2 in a monomeric glucose residue at position 13 or position 19 in a compound represented by Formula (I); and
   (c) a protein including an amino acid sequence having a sequence identity of 90% or more to the amino acid sequence of SEQ ID NO: 2 but in which an amino acid residue corresponding to the amino acid residue at position 155 is the same and having an activity to add hexose at position 2 in the monomeric glucose residue at position 13 or position 19 in the compound of Formula (I): 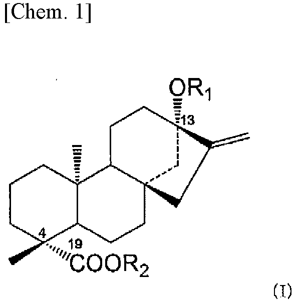
   wherein R₁ and R₂ each independently represent H, a C₁ to C₂₀ alkyl group, a C₂ to C₂₀ alkenyl group, a C₂ to C₂₀ alkynyl group, a C₄ to C₂₀ alkyldienyl group, a C₆ to C₁₈ aryl group, a C₆ to C₂₀ alkylaryl group, a C₆ to C₂₀ arylalkyl group, a C₄ to C₂₀ cycloalkyl group, a C₄ to C₂₀ cycloalkenyl group, a (C₃ to C₁₀ cycloalkyl)C₁ to C₁₀ alkyl group or a sugar residue, with the proviso that R₁ and/or R₂ is a monomeric glucose residue.
[2] The protein according to [1], wherein the amino acid residue at position 42 in the amino acid sequence of SEQ ID NO: 2 or an amino acid residue corresponding thereto is substituted with a phenylalanine residue.
[3] The protein according to [1] or [2], wherein the hexose is glucose or rhamnose.
[4] The protein according to any one of [1] to [3], wherein the compound represented by Formula (I) is steviolmonoside, rubusoside, or stevioside.
[5] A polynucleotide encoding the protein according to any one of [1] to [4].
[6] A vector including the polynucleotide according to [5].
[7] A non-human transformant including the polynucleotide according to [5].
[8] The transformant according to [7], including UGT85C2, UGT76G1, and/or UGT74G1.
[9] An extract of the transformant according to [7] or [8].
[10] A food, a medicament, or an industrial raw material including the extract according to [9].
[11] A method for producing the protein according to any one of [1] to [4], the method including culturing the non-human transformant according to [7] or [8].
[12] A method for producing a steviol glycoside or a steviol glycoside composition, the method including culturing the non-human transformant according to [7] or [8].
[13] A method for producing a steviol glycoside or a steviol glycoside composition, the method including reacting the protein according to any one of [1] to [4], a UDP-sugar, and the compound represented by Formula (I).
[14] The method according to [13], wherein the UDP-sugar is UDP-glucose or UDP-rhamnose.
[15] The method according to any one of [12] to [14], wherein the steviol glycoside composition includes RebE, steviolbioside, stevioside, and/or dulcoside A.

### Advantageous Effects of Invention

According to the present invention, it is possible to provide a glycosyltransferase having high activity to add hexose at position 2 in a monomeric glucose residue at position 13 or position 19 in a steviol glycoside, and a method for producing a steviol glycoside using the enzyme. In addition, it is possible to produce steviol glycosides having good taste such as RebD and RebM in a high yield by combining the protein of the present invention with another glycosyltransferase involved in synthesis of steviol glycosides.

### Description of Embodiments

The present invention will now be described in detail. The following embodiments are provided for illustrating the present invention and are not intended to limit the present invention only thereto. The present invention can be implemented in various embodiments as long as the gist of the present invention is not deviated.

Note that all documents, as well as laid-open application publications, patent application publications, and other patent documents cited herein shall be incorporated herein by reference. In addition, the present specification encompasses the contents described in the specification and drawings of the Japanese patent application (the Japanese Patent Application No. 2022-110052) filed on July 7, 2022, which serves as a basis for claiming priority of the present application.

### 1. Glycosyltransferase

In one aspect, the present invention provides a protein selected from the following (a) to (c) (hereinafter, sometimes referred to as the "protein of the present invention").
(a) A protein including the amino acid sequence of SEQ ID NO: 2;
(b) a protein including an amino acid sequence in which 1 to 48 amino acid residues other than the amino acid residue at position 155 are deleted, substituted, inserted, and/or added in the amino acid sequence of SEQ ID NO: 2, and having an activity to add hexose at position 2 in a monomeric glucose residue at position 13 or position 19 in a compound represented by the following Formula (I); and
(c) a protein including an amino acid sequence having a sequence identity of 90% or more to the amino acid sequence of SEQ ID NO: 2 but in which an amino acid residue corresponding to the amino acid residue at position 155 is the same as the amino acid residue at position 155 of SEQ ID NO: 2 and having an activity to add hexose at position 2 in the monomeric glucose residue at position 13 or position 19 in the compound represented by the following Formula (I): 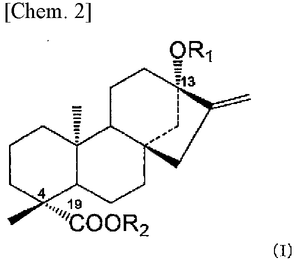
wherein R₁ and R₂ each independently represent H, a C₁ to C₂₀ alkyl group, a C₂ to C₂₀ alkenyl group, a C₂ to C₂₀ alkynyl group, a C₄ to C₂₀ alkyldienyl group, a C₆ to Cis aryl group, a C₆ to C₂₀ alkylaryl group, a C₆ to C₂₀ arylalkyl group, a C₄ to C₂₀ cycloalkyl group, a C₄ to C₂₀ cycloalkenyl group, a (C₃ to C₁₀ cycloalkyl) C₁ to C₁₀ alkyl group or a sugar residue, with the proviso that R₁ and/or R₂ is a monomeric glucose residue.

In a case where the amino acid residue at position 155 in the amino acid sequence of SEQ ID NO: 2 is moved to another position by deletion, insertion, or the like of an amino acid residue, in the protein (c) of the present invention, the amino acid residue corresponding to the amino acid residue at position 155 in the amino acid sequence of SEQ ID NO: 2 means an amino acid residue present at the other position. For example, in a case where the protein (c) of the present invention includes an amino acid sequence in which the amino acid residue at position 30 in the amino acid sequence of SEQ ID NO: 2 is deleted, the "amino acid residue corresponding to the amino acid residue at position 155 in the amino acid sequence of SEQ ID NO: 2" in this protein (c) is the amino acid residue at position 154. Which amino acid residue corresponds to the amino acid residue at position 155 in the amino acid sequence of SEQ ID NO: 2 can be confirmed by, for example, aligning the amino acid sequence of SEQ ID NO: 2 with the amino acid sequence of the protein (c) of the present invention.

In some embodiments, the protein of the present invention includes an amino acid sequence in which the amino acid residue at position 42 in the amino acid sequence of SEQ ID NO: 2 or an amino acid residue corresponding thereto is substituted with a phenylalanine residue (e.g., SEQ ID NO: 4). In a case where the amino acid residue at position 42 in the amino acid sequence of SEQ ID NO: 2 is moved to another position by deletion or insertion of an amino acid residue, in the protein (b) or (c) of the present invention, the amino acid residue corresponding to the amino acid residue at position 42 in the amino acid sequence of SEQ ID NO: 2 means an amino acid residue present at the other position. For example, in a case where the protein (b) of the present invention includes the amino acid sequence in which the amino acid residue at position 30 is deleted in the amino acid sequence of SEQ ID NO: 2, the "amino acid residue corresponding to the amino acid residue at position 42 in the amino acid sequence of SEQ ID NO: 2" in this protein (b) is the amino acid residue at position 41. Which amino acid residue corresponds to the amino acid residue at position 42 in the amino acid sequence of SEQ ID NO: 2 can be confirmed by, for example, aligning the amino acid sequence of SEQ ID NO: 2 with the amino acid sequence of the protein (b) or (c) of the present invention.

In some embodiments, the protein of the present invention includes a protein selected from:
(a') a protein including an amino acid sequence of SEQ ID NO: 4;
(b') a protein including an amino acid sequence in which 1 to 48 amino acid residues other than amino acid residues at position 42 and position 155 are deleted, substituted, inserted, and/or added in the amino acid sequence of SEQ ID NO: 4, and having an activity to add hexose at position 2 in the monomeric glucose residue at position 13 or position 19 in the compound represented by Formula (I) described above, and (c') a protein including an amino acid sequence having a sequence identity of 90% or more to the amino acid sequence of SEQ ID NO: 4 but in which the amino acid residues corresponding to the amino acid residues at position 42 and position 155 are respectively the same as the amino acid residues at position 42 and position 155 of SEQ ID NO: 4 and having an activity to add hexose at position 2 in the monomeric glucose residue at position 13 or position 19 of the compound represented by Formula (I) described above.

The protein of the present invention may be non-naturally occurring. The protein of the present invention can be artificially obtained by site-directed mutagenesis described in, for example, "Sambrook & Russell, Molecular Cloning: A Laboratory Manual Vol. 3, Cold Spring Harbor Laboratory Press 2001", "Ausubel, Current Protocols in Molecular Biology, John Wiley & Sons 1987 to 1997", "Nuc. Acids. Res., 10, 6487 (1982)", "Proc. Natl. Acad. Sci. USA, 79, 6409 (1982)", "Gene, 34, 315 (1985)", "Nuc. Acids. Res., 13, 4431 (1985)", "Proc. Natl. Acad. Sci. USA, 82, 488 (1985)", and the like.

In the present specification, examples of the "protein having an amino acid sequence in which 1 to 48 amino acid residues other than the amino acid residue at position 155 are deleted, substituted, inserted, and/or added in the amino acid sequence of SEQ ID NO: 2 and having an activity to add hexose at position 2 in the monomeric glucose residue at position 13 or position 19 in the compound represented by Formula (I)" include a protein having an amino acid sequence in which 1 to 48, 1 to 47, 1 to 46, 1 to 45, 1 to 44, 1 to 43, 1 to 42, 1 to 41, 1 to 40, 1 to 39, 1 to 38, 1 to 37, 1 to 36, 1 to 35, 1 to 34, 1 to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9 (1 to several), 1 to 8,1 to 7,1 to 6,1 to 5,1 to 4,1 to 3, 1 to 2, or 1 amino acid residue(s) other than the amino acid residue at position 155 is/are deleted, substituted, inserted, and/or added in the amino acid sequence of SEQ ID NO: 2, and having an activity to add hexose at position 2 in the monomeric glucose residue at position 13 or position 19 of the compound represented by Formula (I). In general, the number of deletions, substitutions, insertions and/or additions of amino acid residues is preferably as small as possible.

Examples of such a protein include a protein including an amino acid sequence having a sequence identity of 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more or 99.9% or more to the amino acid sequence of SEQ ID NO: 2 but in which the amino acid residue corresponding to the amino acid residue at position 155 is the same and having the activity to add hexose at position 2 in the monomeric glucose residue at position 13 or position 19 in the compound represented by Formula (I). In general, the larger the numerical value of the sequence identity, the more preferable.

Similarly, in the present specification, examples of the "protein including an amino acid sequence in which 1 to 48 amino acid residues other than the amino acid residues at position 42 and position 155 are deleted, substituted, inserted, and/or added in the amino acid sequence of SEQ ID NO: 4, and having an activity to add hexose at position 2 in the monomeric glucose residue at position 13 or position 19 of the compound represented by Formula (I)" include a protein having an amino acid sequence in which 1 to 48, 1 to 47, 1 to 46, 1 to 45, 1 to 44, 1 to 43, 1 to 42, 1 to 41, 1 to 40, 1 to 39, 1 to 38, 1 to 37, 1 to 36, 1 to 35, 1 to 34, 1 to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20,1 to 19, 1 to 18, 1 to 17, 1 to 16, 1 to 15, 1 to 14, 1 to 13, 1 to 12, 1 to 11, 1 to 10, 1 to 9 (1 to several), 1 to 8, 1 to 7, 1 to 6, 1 to 5, 1 to 4, 1 to 3, 1 to 2, or 1 amino acid residue(s) other than the amino acid residues at positions 42 and 155 is/are deleted, substituted, inserted and/or added in the amino acid sequence of SEQ ID NO: 4, and having an activity to add hexose at position 2 in the monomeric glucose residue at position 13 or position 19 of the compound represented by Formula (I). In general, the number of deletions, substitutions, insertions and/or additions of amino acid residues is preferably as small as possible.

Examples of such a protein include a protein including an amino acid sequence having a sequence identity of 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more to the amino acid sequence of SEQ ID NO: 4 but in which the amino acid residue corresponding to the amino acid residue at position 155 is the same and having the activity to add hexose at position 2 in the monomeric glucose residue at position 13 or position 19 in the compound represented by Formula (I). In general, the larger the numerical value of the sequence identity, the more preferable.

In the present specification, the "C₁ to C₂₀ alkyl group" is preferably a C₁ to C₁₀ alkyl group, and more preferably a C₁ to C₆ alkyl group. Examples of the alkyl group include, but are not limited to, methyl, ethyl, propyl, isopropyl, n-butyl, sec-butyl, tert-butyl, pentyl, hexyl, and dodecanyl.

In the present specification, the "C₂ to C₂₀ alkenyl group" is preferably a C₂ to C₁₀ alkenyl group, and more preferably a C₂ to C₆ alkenyl group. Examples of the alkenyl group include, but are not limited to, vinyl, allyl, propenyl, isopropenyl, 2-methyl-1-propenyl, 2-methylallyl, and 2-butenyl.

In the present specification, the "C₂ to C₂₀ alkynyl group" is preferably a C₂ to C₁₀ alkynyl group, and more preferably a C₂ to C₆ alkynyl group. Examples of the alkynyl group include, but are not limited to, ethynyl, 2-propynyl, and 2-butynyl.

In the present specification, the "C₄ to C₂₀ alkyldienyl group" is preferably a C₄ to C₁₀ alkyldienyl group, and more preferably a C₄ to C₆ alkyldienyl group. Examples of the alkyldienyl group include, but are not limited to, 1,3-butadienyl.

In the present specification, the "C₆ to C₁₈ aryl group" is preferably a C₆ to C₁₀ aryl group. Examples of the aryl group include, but are not limited to, phenyl, 1-naphthyl, 2-naphthyl, indenyl, biphenylyl, anthryl, and phenanthryl.

In the present specification, the "C₆ to C₂₀ alkylaryl group" is preferably a C₆ to C₁₂ alkylaryl group. Examples of the alkylaryl group include, but are not limited to, o-tolyl, m-tolyl, p-tolyl, 2,3-xylyl, 2,4-xylyl, 2,5-xylyl, o-cumenyl, m-cumenyl, p-cumenyl, and mesityl.

In the present specification, the "C₆ to C₂₀ arylalkyl group" is preferably a C₆ to C₁₂ arylalkyl group. Examples of the arylalkyl group include, but are not limited to, benzyl, phenethyl, diphenylmethyl, triphenylmethyl, 1-naphthylmethyl, 2-naphthylmethyl, 2,2-diphenylethyl, 3-phenylpropyl, 4-phenylbutyl, and 5-phenylpentyl.

In the present specification, the "C₄ to C₂₀ cycloalkyl group" is preferably a C₄ to C₁₀ cycloalkyl group. Examples of the cycloalkyl group include, but are not limited to, cyclopropyl, cyclobutyl, cyclopentyl, and cyclohexyl.

In the present specification, the "C₄ to C₂₀ cycloalkenyl group" is preferably a C₄ to C₁₀ cycloalkenyl group. Examples of the cycloalkenyl group include, but are not limited to, cyclopropenyl, cyclobutenyl, 2-cyclopenten-1-yl, 2-cyclohexen-1-yl, and 3-cyclohexen-1-yl.

In the present specification, examples of the "(C₃ to C₁₀ cycloalkyl) C₁ to C₁₀ alkyl group" include, but are not limited to, methylcyclopropyl, ethylcyclopropyl, methylcyclobutyl, ethylcyclopentyl, and methylcyclohexyl.

In the present specification, the "sugar residue" is not particularly limited and may be a residue of a sugar including one or more pentoses, hexoses (including deoxyhexoses) or combinations thereof.
Examples of the pentoses include ribose, arabinose, and lyxose, and examples of the hexose include allose, altrose, glucose, mannose, gulose, idose, galactose, talose, and rhamnose.

Preferably, the "sugar residue" is a residue of a sugar including one or more hexose units, and more preferably a sugar residue of a glucose monomer (-Glc) or a glucose dimer (-Glc-Glc). In the sugar residues of the glucose dimer, the glucose residues are preferably linked to each other by a β2,1 glycosidic bond.

Preferably, the compound of Formula (I) is steviolmonoside, rubusoside, or stevioside.

The hexose to be added to position 2 of the monomeric glucose residue at position 13 or position 19 of the compound represented by Formula (I) by the protein of the present invention is not particularly limited, but is preferably a hexose selected from glucose, rhamnose, mannose, and galactose, and more preferably glucose or rhamnose. Most preferably, the hexose is glucose.

The activity to add hexose at position 2 in the monomeric glucose residue at position 13 or position 19 of the compound represented by Formula (I) can be verified by, for example, incubation at 20 to 40°C for 10 minutes to 2 hours in a buffer solution (e.g., sodium phosphate buffer or potassium phosphate buffer) in the neutral region of pH 6.0 to 8.0 containing a test protein, a UDP sugar (e.g., UDP-glucose) at 1 to 1000 µM (preferably 100 to 700 µM, most preferably 500 µM), and a substrate compound (compound of Formula (I)) at 1 to 500 µM (preferably 100 to 500 µM, most preferably 250 µM), purifying the reaction liquid as necessary, and analyzing the purified reaction liquid by a known method such as HPLC analysis or liquid chromatography-mass spectrometry (LC-MS) analysis.

Alternatively, the activity to add hexose at position 2 in the monomeric glucose residue at position 13 or position 19 of the compound represented by Formula (I) can also be verified by, for example, culturing a transformant (e.g., a yeast) co-expressing a test protein and UGT85C2 in a glucose-containing medium (e.g., for 2 to 48 hours, preferably 3 to 24 hours, most preferably overnight), then culturing the transformant in a medium containing steviol (e.g., 1 to 100 µg/mL, preferably 2 to 50 µg/mL, most preferably 10 µg/mL) for a predetermined time (e.g., 3 to 72 hours, preferably 6 to 48 hours, most preferably two nights), and analyzing the culture supernatant by a known method such as HPLC analysis or LC-MS analysis.

In a case where a compound (e.g., steviolbioside, stevioside, dulcoside A, RebE, RebC, or the like) in which a hexose is added to position 2 of the monomeric glucose residue at position 13 or position 19 of the compound represented by Formula (I) is detected as a result of the analysis, it can be said that the test protein has an activity to add hexose at position 2 in the monomeric glucose residue at position 13 or position 19 of the compound represented by Formula (I).

The protein of the present invention is more active in generating RebE than known proteins (e.g., UGT91D-like3 (SEQ ID NO: 6)) in a case where combined with UGT85C2 and using steviol as a substrate. The height of activity for UGT91D-like3 may be, for example, 1.2 times or more, 1.4 times or more, 1.6 times or more, 1.8 times or more, 2 times or more, 2.2 times or more, 2.4 times or more, 2.6 times or more, 2.8 times or more, 3 times or more, 3.5 times or more, or 4 times or more in terms of the amount of RebE generated.

The protein of the present invention also has higher activity of generating RebD and RebM in addition to RebE than known proteins (e.g., UGT91D-like3) in a case where it is used in combination with UGT74G1, UGT76G1, and UGT85C2 and using steviol as a substrate. The height of activity for UGT91D-like3 may be, for example, 1.2 times or more, 1.4 times or more, 1.6 times or more, 1.8 times or more, 2 times or more, 2.2 times or more, 2.4 times or more, 2.5 times or more, 2.6 times or more, 2.8 times or more, 3 times or more, 3.2 times or more, 3.4 times or more, or 3.6 times or more in terms of the amount of RebD produced, and 1.2 times or more, 1.4 times or more, 1.6 times or more, 1.8 times or more, 1.9 times or more, 2 times or more, 2.2 times or more, 2.4 times or more, 2.6 times or more, 2.8 times or more, or double or more, in terms of the amount of RebM generated.

The expression "one or more amino acid residues are deleted, substituted, inserted, and/or added in the amino acid sequence of the protein of the present invention" means that one or more amino acid residues are deleted, substituted, inserted, and/or added at any one or more positions in the same sequence, and two or more kinds of deletion, substitution, insertion, and addition may occur simultaneously.

Hereinafter, examples of mutually substitutable amino acid residues will be indicated. Amino acid residues included in the same group can be substituted with each other. Group A: leucine, isoleucine, norleucine, valine, norvaline, alanine, 2-aminobutanoate, methionine, o-methylserine, t-butylglycine, t-butylalanine, and cyclohexylalanine; Group B: aspartic acid, glutamic acid, isoaspartic acid, isoglutamic acid, 2-aminoadipic acid, and 2-aminosuberic acid; Group C: asparagine, and glutamine; Group D: lysine, arginine, ornithine, 2,4-diaminobutanoic acid, and 2,3-diaminopropionic acid; Group E: proline, 3-hydroxyproline, and 4-hydroxyproline; Group F: serine, threonine, and homoserine; Group G: phenylalanine, and tyrosine.

The protein of the present invention can be obtained by expressing a polynucleotide encoding the protein (see "polynucleotide of the present invention" described below) in an appropriate host cell, but can also be produced by a chemical synthesis method such as a fluorenylmethyloxycarbonyl method (Fmoc method) or a t-butyloxycarbonyl method (tBoc method). Alternatively, the protein can be chemically synthesized using a peptide synthesizer available from Advanced Automation Peptide Protein Technologies, Perkin Elmer, Protein Technologies, PerSeptive, Applied Biosystems, Shimadzu Corporation, or the like.

### 2. Method for producing steviol glycoside

Steviol glycosides can be produced easily and in large amounts by utilizing the activity, which the protein of the present invention has, to add hexose at position 2 in the monomeric glucose residue at position 13 or position 19 of the compound represented by Formula (I).

Thus, in another aspect, the present invention provides a method for producing a steviol glycoside or a steviol glycoside composition (hereinafter, sometimes referred to as "production method A of the present invention"), the method including reacting the protein of the present invention, an UDP-sugar, and the compound represented by Formula (I) described above.

In the present specification, the "UDP-sugar" is a uridine diphosphate (UDP)-linked sugar. Preferred examples of the sugar moiety in the UDP-sugar include a sugar including one or more hexoses. Examples of the hexose are described above. Preferably, the UDP-sugar is a UDP-hexose, more preferably selected from the group consisting of UDP-glucose, UDP-rhamnose, UDP-mannose, and UDP-galactose. Most preferably, the UDP-sugar is UDP-glucose.

The production method A of the present invention includes reacting the protein of the present invention, the UDP sugar, and the compound represented by Formula (I) (step 1). In step 1, a hexose is added to position 2 of the monomeric glucose residue at position 13 or position 19 of the compound represented by Formula (I). The production method A of the present invention may further include purifying the steviol glycoside generated in step 1 (step 2). The production method A of the present invention may further include further adding a sugar to the steviol glycoside generated in step 1 (step 3). Step 3 may include, for example, allowing another enzyme involved in biosynthesis of a steviol glycoside, such as UGT74G1, UGT76G1, or UGT85C2, to act on the steviol glycoside generated in step 1. The production method A of the present invention may further include generating the compound represented by Formula (I) from steviol (step 4). Step 4 may include, for example, allowing another enzyme involved in biosynthesis of a steviol glycoside, such as UGT74G1, UGT76G1, or UGT85C2, to act on the steviol glycoside generated in step 1.

Examples of the steviol glycoside generated by the production method A of the present invention include, but are not limited to, steviolbioside, stevioside, dulcoside A, RebE, and RebC. The steviol glycoside composition refers to a composition containing at least one steviol glycoside generated by the production method A of the present invention, and contains, for example, at least one, preferably at least two of steviolbioside, stevioside, dulcoside A, RebE, and RebC. The steviol glycoside composition may include a compound used as a substrate, for example, steviol, steviolmonoside, rubusoside, stevioside, or the like.

As for the ratio of steviol glycosides contained in the steviol glycoside composition, on a mass basis, in a case of being combined with at least UGT85C2, RebE/stevioside may be, for example, 0.05 to 5.0, 0.10 to 4.5, 0.15 to 4.0, 0.20 to 3.5, 0.25 to 3.0, 0.30 to 2.5, 0.35 to 2.0, 0.40 to 1.5, 0.45 to 1.0, 0.47 to 0.8, or 0.48 to 0.6, and particularly about 0.5. In a case of being combined with at least UGT74G1, UGT76G1, and UGT85C2, RebD/RebA may be, for example, 0.025 to 2.50, 0.050 to 2.25, 0.075 to 2.0, 0.10 to 1.75, 0.125 to 1.50, 0.150 to 1.25, 0.175 to 1.0, 0.20 to 0.75, 0.21 to 0.50, 0.22 to 0.40, or 0.23 to 0.30, and particularly about 0.25, RebD/RebI may be, for example, 0.075 to 7.5, 0.150 to 6.75, 0.225 to 6.0, 0.30 to 5.25, 0.375 to 4.5, 0.450 to 3.75, 0.525 to 3.0, 0.60 to 2.25, 0.675 to 1.5, 0.70 to 1.0, or 0.725 to 0.8, and particularly about 0.75, RebD/stevioside may be, for example, 0.25 to 25.0, 0.50 to 22.5, 0.75 to 20.0, 1.0 to 17.5, 1.25 to 15.0, 1.50 to 12.5, 1.75 to 10.0, 2.0 to 7.5, 2.1 to 5.0, 2.2 to 4.0, or 2.3 to 3.00, and particularly about 2.5, RebM/RebA may be, for example, 0.05 to 5.0, 0.10 to 4.5, 0.15 to 4.0, 0.20 to 3.5, 0.25 to 3.0, 0.30 to 2.5, 0.35 to 2.0, 0.40 to 1.5, 0.45 to 1.0, 0.47 to 0.8, or 0.48 to 0.6, particularly about 0.5, RebM/RebI may be, for example, 0.18 to 18, 0.28 to 16, 0.38 to 14, 0.58 to 12, 0.78 to 10, 0.98 to 8, 1.18 to 6, 1.38 to 5, 1.58 to 4, 1.78 to 3, or 1.6 to 2, and particularly about 1.8, RebM/stevioside may be, for example, 0.5 to 50, 1.0 to 45, 1.5 to 40, 2.0 to 35, 2.5 to 30, 3.0 to 25, 3.5 to 20, 4.0 to 15, 4.5 to 10, 4.7 to 8, or 4.8 to 6,, and particularly about 5, (RebD + RebM)/RebA may be, for example, 0.075 to 7.5, 0.150 to 6.75, 0.225 to 6.0, 0.30 to 5.25, 0.375 to 4.5, 0.450 to 3.75, 0.525 to 3.0, 0.60 to 2.25, 0.675 to 1.5, 0.70 to 1.0, or 0.725 to 0.8, and particularly about 0.75, (RebD + RebM)/RebI may be, for example, 0.25 to 25.0, 0.50 to 22.5, 0.75 to 20.0, 1.0 to 17.5, 1.25 to 15.0, 1.50 to 12.5, 1.75 to 10.0, 2.0 to 7.5, 2.1 to 5.0, 2.2 to 4.0, or 2.3 to 3.00, and particularly about 2.5, (RebD + RebM)/stevioside may be, for example, 0.75 to 75.0, 1.50 to 67.5, 2.25 to 60.0, 3.0 to 52.5, 3.75 to 45.0, 4.5 to 37.5, 5.25 to 30.0, 6.0 to 22.5, 6.75 to 15.0, 7.0 to 10.0, or 7.25 to 8.0, and particularly about 7.5, RebE/RebA may be, for example, 0.005 to 0.50, 0.010 to 0.45, 0.015 to 0.40, 0.020 to 0.35, 0.025 to 0.30, 0.030 to 0.25, 0.035 to 0.20, 0.040 to 0.15, 0.045 to 0.10, 0.047 to 0.08, or 0.048 to 0.06, and particularly about 0.05, RebE/RebI may be, for example, 0.025 to 2.50, 0.050 to 2.25, 0.075 to 2.0, 0.10 to 1.75, 0.125 to 1.50, 0.150 to 1.25, 0.175 to 1.0, 0.20 to 0.75, 0.21 to 0.50, 0.22 to 0.40, or 0.23 to 0.30, and particularly about 0.25, and RebE/stevioside may be, for example, 0.05 to 5.0, 0.10 to 4.5, 0.15 to 4.0, 0.20 to 3.5, 0.25 to 3.0, 0.30 to 2.5, 0.35 to 2.0, 0.40 to 1.5, 0.45 to 1.0, 0.47 to 0.8, or 0.48 to 0.6, and particularly about 0.5.

The steviol glycosides generated can be purified by a known method such as extraction with a suitable solvent (aqueous solvent such as water, or organic solvent such as alcohol, ether, and acetone), ethyl acetate or other organic solvent: water gradient, HPLC, gas chromatography, time-of-flight mass spectrometry (TOF-MS), or ultra-high performance liquid chromatography (UPLC).

### 3. Non-human transformant expressing protein of present invention

The steviol glycoside can also be generated in cells of a microorganism (bacteria such as Escherichia coli, fungi such as yeast, or the like), a plant, an insect, a mammal other than human, or the like using the protein of the present invention. In this case, the steviol glycoside can be generated by introducing a polynucleotide encoding the protein of the present invention into a host cell derived from a microorganism, a plant, an insect, a mammal other than human, or the like to express the protein of the present invention, and reacting the protein of the present invention with the UDP-sugar and the compound represented by Formula (I) present in the cell.

Thus, the present invention provides a non-human transformant (hereinafter, referred to as the "transformant of the present invention") including a polynucleotide encoding the protein of the present invention (hereinafter, sometimes referred to as the "polynucleotide of the present invention").

In some embodiments, the polynucleotide of the present invention includes a polynucleotide selected from:
(a) a polynucleotide including the nucleotide sequence of SEQ ID NO: 1;
(b) a polynucleotide encoding a protein including the amino acid sequence of SEQ ID NO: 2;
(c) a polynucleotide encoding a protein including an amino acid sequence in which 1 to 48 amino acid residues other than the amino acid residue at position 155 are deleted, substituted, inserted, and/or added in the amino acid sequence of SEQ ID NO: 2 and having an activity to add hexose at position 2 in a monomeric glucose residue at position 13 or position 19 in the compound represented by Formula (I) described above;
(d) a polynucleotide encoding a protein including an amino acid sequence having a sequence identity of 90% or more to the amino acid sequence of SEQ ID NO: 2 but in which the amino acid residue corresponding to the amino acid residue at position 155 is the same and having an activity to add hexose at position 2 in the monomeric glucose residue at position 13 or position 19 in the compound represented by Formula (I) described above; and
(e) a polynucleotide hybridizing under highly stringent conditions with a polynucleotide including a nucleotide sequence complementary to the nucleotide sequence of the polynucleotide of (a) or (b) described above but encoding a protein which includes an amino acid sequence in which the amino acid residue corresponding to the amino acid residue at position 155 of SEQ ID NO: 2 is the same as the amino acid residue at position 155 of SEQ ID NO: 2, and has the activity to add hexose at position 2 in the monomeric glucose residue at position 13 or position 19 in the compound represented by Formula (I) described above.

In some embodiments, the polynucleotide of the present invention includes a polynucleotide selected from:
(a') a polynucleotide including the nucleotide sequence of SEQ ID NO: 3;
(b') a polynucleotide encoding a protein including the amino acid sequence of SEQ ID NO: 4;
(c') a polynucleotide encoding a protein including an amino acid sequence in which 1 to 48 amino acid residues other than the amino acid residues at position 42 and position 155 are deleted, substituted, inserted, and/or added in the amino acid sequence of SEQ ID NO: 4 and having an activity to add hexose at position 2 in the monomeric glucose residue at position 13 or position 19 in the compound represented by Formula (I) described above;
(d') a polynucleotide encoding a protein including an amino acid sequence having 90% or more sequence identity to the amino acid sequence of SEQ ID NO: 4 but in which amino acid residues corresponding to the amino acid residues at positions 42 and 155 are the same and having an activity to add hexose at position 2 in the monomeric glucose residue at position 13 or position 19 in the compound represented by Formula (I) described above; and
(e') a polynucleotide hybridizing under highly stringent conditions with a polynucleotide including a nucleotide sequence complementary to the nucleotide sequence of the polynucleotide of (a') or (b') described above, but encoding a protein which includes an amino acid sequence in which the amino acid residues corresponding to the amino acid residues at positions 42 and 155 of SEQ ID NO: 4 are respectively the same as the amino acid residues at positions 42 and 155 of SEQ ID NO: 4, and which has the activity to add hexose at position 2 in the monomeric glucose residue at position 13 or position 19 in the compound represented by Formula (I) described above.

As used herein, the term "polynucleotide" refers to DNA or RNA.

In the present specification, the expression "polynucleotide hybridizing under highly stringent conditions" refers to a polynucleotide obtained by using, for example, all or a part of a polynucleotide including a nucleotide sequence complementary to the nucleotide sequence of SEQ ID NO: 1 as a probe in a colony hybridization method, a plaque hybridization method, a Southern hybridization method, or the like. As a hybridization method, for example, methods described in "Sambrook & Russell, Molecular Cloning: A Laboratory Manual Vol. 3, Cold Spring Harbor, Laboratory Press 2001", "Ausubel, Current Protocols in Molecular Biology, John Wiley & Sons 1987 to 1997", and the like can be used.

In the present specification, examples of the "highly stringent conditions" include, but are not limited to, conditions of (1) 5 × SSC, 5 × Denhardt's solution, 0.5% SDS, and 50% formamide, at 50°C, (2) 0.2 × SSC, and 0.1% SDS, at 60°C, (3) 0.2 × SSC, and 0.1% SDS, at 62°C, (4) 0.2 × SSC, and 0.1% SDS, at 65°C, or (5) 0.1 × SSC, and 0.1% SDS, at 65°C. Under these conditions, it can be expected that a DNA having higher sequence identity can be obtained more efficiently as the temperature is elevated. However, a plurality of factors such as a temperature, a probe concentration, a probe length, an ionic strength, a time, and a salt concentration can be considered as factors affecting the stringency of hybridization, and those skilled in the art can realize the same stringency by appropriately selecting these factors.

Note that in a case where a commercially available kit is used for hybridization, for example, Alkphos Direct Labelling and Detection System (GE Healthcare) can be used. In this case, according to the protocol attached to the kit, after incubation with a labeled probe overnight, a membrane is washed with a primary washing buffer containing 0.1% (w/v) SDS under the condition of 55 to 60°C, and then, a hybridized DNA can be detected. Alternatively, in a case where a probe is labeled with digoxigenin (DIG) using a commercially available reagent (for example, PCR labeling mix (Roche Diagnostics) or the like) in preparing the probe, hybridization can be detected using a DIG nucleic acid detection kit (Roche Diagnostics).

In addition to above-described, examples of the hybridizable polynucleotide include a polynucleotide having 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more sequence identity to the polynucleotide of SEQ ID NO: 1 or 3 or the polynucleotide encoding the amino acid sequence of SEQ ID NO: 2 or 4 when calculated by homology search software such as FASTA or BLAST using default parameters.

Note that the sequence identity of an amino acid sequence or a nucleotide sequence can be determined using FASTA (Science 227 (4693): 1435 to 1441, (1985)) or Basic Local Alignment Search Tool (BLAST), which is an algorithm by Karlin and Altschul (Proc. Natl. Acad. Sci. USA 872264 to 2268, 1990; Proc Natl Acad Sci USA 90: 5873, 1993). Programs called blastn, blastx, blastp, tblastn and tblastx based on the BLAST algorithm have been developed (Altschul SF, et al: J Mol Biol 215: 403, 1990). In a case where a nucleotide sequence is analyzed using blastn, parameters are set to, for example, score = 100 and wordlength = 12. In a case where an amino acid sequence is analyzed using blastp, parameters are set to, for example, score = 50 and wordlength = 3. In a case where BLAST and Gapped BLAST programs are used, default parameters of each program are used.

The polynucleotide of the present invention described above can be obtained by a known genetic engineering technique or a known synthesis technique.

The polynucleotide of the present invention is preferably introduced into a host in a state of being inserted into an appropriate expression vector.

A suitable expression vector typically includes:
(i) a promoter capable of transcription in a host cell;
(ii) the polynucleotide of the present invention bound to the promoter; and
(iii) an expression cassette including, as a constituent, a signal that functions in the host cell for transcription termination and polyadenylation of an RNA molecule.

Examples of a method for preparing an expression vector include, but are not particularly limited to, a method using a plasmid, a phage, a cosmid, or the like.

A specific kind of the vector is not particularly limited, and a vector that can be expressed in a host cell may be appropriately selected. For example, to express the polynucleotide of the present invention, a promoter sequence is appropriately selected depending on a kind of a host cell, and a vector in which the promoter sequence and the polynucleotide of the present invention are incorporated into various plasmids or the like can be used as an expression vector.

The expression vector of the present invention includes an expression control region (for example, a promoter, a terminator, and/or a replication origin) depending on the kind of the host to which the expression vector is to be introduced. Examples of a promoter for a bacterium include a trc promoter, a tac promoter, and a lac promoter; examples of a promoter for a yeast include a GAL1 promoter, a GAL10 promoter, a glyceraldehyde-3-phosphate dehydrogenase promoter, and a PH05 promoter; and examples of a promoter for a filamentous fungus include an amylase promoter, and a trpC promoter. Examples of a promoter for a plant include a 35S RNA promoter of cauliflower mosaic virus, an rd29A gene promoter, an rbcS promoter, and a mac-1 promoter in which an enhancer sequence of the 35S RNA promoter of cauliflower mosaic virus is added to the 5' side of a mannopine synthase promoter sequence derived from Agrobacteria. Examples of a promoter for an animal include viral promoters (e.g., an SV40 early promoter, an SV40 late promoter, and the like).

Preferably, the expression vector includes at least one selectable marker. As such a marker, auxotrophic markers (ura5, niaD, TRP1, URA3, HIS3, and LEU2), drug-resistance markers (hygromycin, zeocin, and G418), copper-resistance genes (CUP1), cerulenin-resistance genes (mutant FAS2, and PDR4) (Aritomi et al., Biosci Biotechnol Biochem. 2004; 68 (1): 206 to 14; Hussain et al., Gene, vol. 101, p. 149, 1991, respectively), and the like can be used.

A method for preparing the transformant of the present invention is not particularly limited, and examples thereof include a method in which an expression vector containing the polynucleotide of the present invention is introduced into a host for transformation.

A host cell to be used for transformation is not particularly limited, and various known cells can be suitably used. Examples of the host cell include bacteria such as Escherichia coli, yeasts (budding yeast Saccharomyces cerevisiae, fission yeast Schizosaccharomyces pombe, and the like), plant cells, and animal cells other than human. The host cell may be a heterologous cell (a cell derived from a species other than stevia) or a homologous cell (a cell derived from stevia). In a case where a homologous cell is used as a transformant, the transformant of the present invention expresses the exogenously introduced protein of the present invention in addition to the endogenously expressed protein, and thus an enzymatic reaction, particularly biosynthesis of a steviol glycoside, is promoted as compared with a wild-type stevia cell.

The host cell may be a cell capable of generating the compound represented by Formula (I). Here, the host cell is not limited to a cell capable of generating the compound represented by Formula (I) in a natural state, and may be, for example, a cell into which a known gene necessary for generation of the compound represented by Formula (I) has been introduced.

Examples of a known gene encoding an enzyme contributing to synthesis of the compound represented by Formula (I) include, but are not limited to, UGT85C2, UGT74G1, and UGT76G1 (Non-Patent Literature 2).

In a case where the host cell is a cell that cannot produce the compound represented by Formula (I), a steviol glycoside can be produced without introducing a gene encoding an enzyme contributing to the synthesis of the compound represented by Formula (I) by adding the compound represented by Formula (I) or a plant extract containing the compound as a substrate to a culture system of a transformant obtained by introducing the gene of the present invention into the host cell.

Furthermore, a more highly glycosylated steviol glycoside (e.g., RebA, RebB, RebD, RebI, RebM, RebQ, or the like) can be prepared by using a host cell into which a gene encoding a glycosyltransferase involved in synthesis of a steviol glycoside has been introduced and expressing the polynucleotide of the present invention in the host cell. Examples of a glycosyltransferase involved in synthesis of a steviol glycosylation enzyme include UGT74G1 (CDS: SEQ ID NO: 7, amino acid sequence: SEQ ID NO: 8), UGT76G1 (CDS: SEQ ID NO: 9, amino acid sequence: SEQ ID NO: 10), and UGT85C2 (CDS: SEQ ID NO: 11, amino acid sequence: SEQ ID NO: 12).

Appropriate culture media and conditions for the above-described host cell are well known in the art. An organism to be transformed is not particularly limited, and examples thereof include various microorganisms, plants, and animals other than human as exemplified above for the host cell.

In addition, general molecular biological techniques can be referred to "Sambrook & Russell, Molecular Cloning: A Laboratory Manual Vol. 3, Cold Spring Harbor Laboratory Press 2001", "Methods in Yeast Genetics, A laboratory manual (Cold Spring Harbor Laboratory Press, Cold Spring Harbor, NY)", and the like.

When the transformant of the present invention is cultured, the protein of the present invention can be produced in the transformant of the present invention. Accordingly, the present invention provides a method for producing the protein of the present invention (method for producing a protein of the present invention), which includes using the transformant of the present invention. The method for producing a protein of the present invention includes culturing the transformant of the present invention. Suitable culture media and conditions are well known in the art. The method for producing a protein of the present invention may further includes recovering the protein of the present invention after culturing the transformant of the present invention. The protein can be recovered by any known technique. The present invention also provides the protein of the present invention produced by the method for producing a protein of the present invention.

In addition, when the transformant is cultured, it is possible to cause the transformant to produce a steviol glycoside. As described above, the production of a steviol glycoside can also be promoted by adding the compound of Formula (I) or a plant extract containing the compound as a substrate to the culture system of the transformant. The steviol glycoside of interest can be obtained by purifying the steviol glycoside accumulated in the transformant and/or secreted out of the transformant (e.g., into the culture).

Accordingly, the present invention provides a method for producing a steviol glycoside or a steviol glycoside composition (production method B of a steviol glycoside of the present invention), in which the transformant of the present invention is used. Suitable culture media and conditions are well known in the art. In addition, the kind, production method, and the like of a steviol glycoside are as described above for the production method A of a steviol glycoside of the present invention. The present invention further provides a steviol glycoside or a steviol glycoside composition produced by the production method B of a steviol glycoside of the present invention.

Any yeast can be used as the host cell for transformation. Specific examples thereof include, but are not limited to, a yeast of the genus Saccharomyces.

As a method for transforming a yeast, a commonly used known method can be used. Examples thereof include, but are not limited to, an electroporation method (Meth. Enzym., 194, p182 (1990)), a spheroplast method (Proc. Natl. Acad. Sci. USA, 75 p1929 (1978)), a lithium acetate method (J. Bacteriology, 153, p163 (1983)), Proc. Natl. Acad. Sci. USA, 75 p1929 (1978), and Methods in yeast genetics, 2000 Edition: A Cold Spring Harbor Laboratory Course Manual. A transformant strain can be selected and obtained as a strain that grows in a medium (for example, an antibiotic-containing medium or a nutrient-deficient medium) having a selection pressure tailored to the selectable marker used.

The transformant may be a plant transformant. The plant transformant can be obtained by introducing an expression vector containing the polynucleotide of the present invention into a plant in such a manner that a polypeptide encoded by the polynucleotide can be expressed. Alternatively, when the gene of the present invention is introduced to be inherited by progeny, a new plant body having the gene can be obtained by using the transformant of the present invention as a mating parent.

The expression vector to be used for transformation of a plant body is not particularly limited as long as the polynucleotide according to the present invention can be expressed in the plant. Examples of such a vector include a vector having a promoter that constitutively expresses the polynucleotide in a plant cell and a vector having a promoter that is inducibly activated by an external stimulus.

Examples of the promoter that constitutively expresses the polynucleotides in a plant cell include a 35S RNA promoter of cauliflower mosaic virus, an rd29A gene promoter, an rbcS promoter, and a mac-1 promoter.

Examples of the promoter that is inducibly activated by an external stimulus include a mouse mammary tumor virus (MMTV) promoter, a tetracycline-responsive promoter, a metallothionein promoter, and a heat shock protein promoter.

The plant to be transformed in the present invention means any of a whole plant, organs of plants (e.g. leaves, petals, stems, roots, seeds, etc.), tissues of plants (e.g. epidermis, phloem, parenchyma, xylem, vascular bundle, palisade tissue, spongiform tissue, etc.), plant cultured cells, and various forms of plant cells (e.g. suspension-cultured cells), protoplasts, leaf sections, calluses, and the like. The plant to be used for transformation is not particularly limited, and may be any of a plant belonging to the class Liliopsida or the class dicotyledon.

For introduction of a gene into a plant, a transformation method known to those skilled in the art (for example, an Agrobacterium method, a gene gun method, a PEG method, an electroporation method, a particle gun method, or the like) is used.

The cell or plant tissue into which the gene has been introduced can be selected by, for example, drug resistance such as hygromycin resistance, and then regenerated into a plant body by a common method. Regeneration of a plant body from a transformed cell can be performed by a method known to those skilled in the art depending on the kind of a plant cell.

Whether or not the polynucleotide of the present invention has been introduced into a plant can be confirmed by detecting the polynucleotide of the present invention by a PCR method, a Southern hybridization method, a Northern hybridization method, or the like, or by confirming the expression of a selectable marker by a predetermined technique (for example, culture in a medium supplemented with a resistance agent, culture in a medium deficient in a specific nutrient, or the like).

Once a transformed plant body in which the polynucleotide of the present invention is incorporated into the genome is obtained, progeny can be obtained by sexual or asexual reproduction of the plant body. In addition, seeds, fruits, cuttings, tubers, tuberous roots, stocks, calluses, protoplasts, and the like can be obtained from the plant body or its progeny, or clones thereof, and the plant body can be mass-produced based on the seeds, fruits, cuttings, tubers, tuberous roots, stocks, calluses, protoplasts, and the like. Thus, the present invention also includes a plant body into which the polynucleotide according to the present invention has been introduced in an expressible manner, progeny of the plant body having the same properties as the plant body, or a tissue derived therefrom.

Transformation methods for various plants have already been reported. The transformant plant according to the present invention is not limited, but as a particularly preferred example, it is desirable to use a plant known to biosynthesize various glycosides using steviol as an aglycone, and examples of such a plant include stevia and Rubus suavissimus.

In a case where a plant transformed with the polynucleotide of the present invention (hereinafter, referred to as the "plant of the present invention" or the "plant body of the present invention") has an appropriate substrate, or an appropriate substrate is added from the outside, the plant can produce a larger amount of steviol glycosides than a wild-type plant.

A complete plant body of the plant of the present invention can be easily obtained by growing seeds, cuttages, bulbs, or the like of the plant of the present invention.

Thus, the plant of the present invention includes an entire plant body, organs of plants (e.g. leaves, petals, stems, roots, seeds, bulbs, etc.), tissues of plants (e.g. epidermis, phloem, parenchyma, xylem, vascular bundle, palisade tissue, spongiform tissue, etc.), plant cultured cells, various forms of plant cells (e.g. suspension-cultured cells), protoplasts, leaf sections, calluses, and the like.

The transformant of the present invention includes processed products of the transformant, for example, a dried product, a lyophilized product, and a disrupted product. Non-limiting examples of the processed products of the transformant include crushed products of transformed cells (e.g., crushed products of a transformed yeast), and dried leaves of transformed plants (e.g., dried leaves of a transformed stevia plant).

In addition, the transformant of the present invention containing a steviol glycoside (including a dried product, a lyophilized product, a crushed product, and the like thereof) itself can be used as a raw material for foods, medicaments, industrial raw materials, or the like. In a case where the transformant of the present invention contains a steviol glycoside, the protein of the present invention is expressed and hexose is added to position 2 of the monomeric glucose residue at position 13 or position 19 of the steviol glycoside. Thus, in a case where all steviol glycosides contained in the transformant are collectively considered, a proportion of steviol glycosides in which a hexose is added to position 2 of the monomeric glucose residue at position 13 or position 19 in the steviol glycoside is naturally higher than in an organism (e.g., a wild-type stevia plant) in which the protein of the present invention is not expressed. Change in such a proportion will affect characteristics of the transformant, for example, sensory characteristics such as sweetness.

As for the ratio of steviol glycosides contained in the steviol glycoside-containing transformant of the present invention, on a generation amount basis, in a transformant co-expressing at least UGT85C2, RebE/stevioside may be, for example, 0.05 to 5.0, 0.10 to 4.5, 0.15 to 4.0, 0.20 to 3.5, 0.25 to 3.0, 0.30 to 2.5, 0.35 to 2.0, 0.40 to 1.5, 0.45 to 1.0, 0.47 to 0.8, or 0.48 to 0.6, and particularly about 0.5. In a transformant co-expressing at least UGT74G1, UGT76G1, and UGT85C2, RebD/RebA may be, for example, 0.025 to 2.50, 0.050 to 2.25, 0.075 to 2.0, 0.10 to 1.75, 0.125 to 1.50, 0.150 to 1.25, 0.175 to 1.0, 0.20 to 0.75, 0.21 to 0.50, 0.22 to 0.40, or 0.23 to 0.30, and particularly about 0.25, RebD/RebI may be, for example, 0.075 to 7.5, 0.150 to 6.75, 0.225 to 6.0, 0.30 to 5.25, 0.375 to 4.5, 0.450 to 3.75, 0.525 to 3.0, 0.60 to 2.25, 0.675 to 1.5, 0.70 to 1.0, or 0.725 to 0.8, and particularly about 0.75, RebD/stevioside may be, for example, 0.25 to 25.0, 0.50 to 22.5, 0.75 to 20.0, 1.0 to 17.5, 1.25 to 15.0, 1.50 to 12.5, 1.75 to 10.0, 2.0 to 7.5, 2.1 to 5.0, 2.2 to 4.0, or 2.3 to 3.00, and particularly about 2.5, RebM/RebA may be, for example, 0.05 to 5.0, 0.10 to 4.5, 0.15 to 4.0, 0.20 to 3.5, 0.25 to 3.0, 0.30 to 2.5, 0.35 to 2.0, 0.40 to 1.5, 0.45 to 1.0, 0.47 to 0.8, or 0.48 to 0.6, particularly about 0.5, RebM/RebI may be, for example, 0.18 to 18, 0.28 to 16, 0.38 to 14, 0.58 to 12, 0.78 to 10, 0.98 to 8, 1.18 to 6, 1.38 to 5, 1.58 to 4, 1.78 to 3, or 1.6 to 2, and particularly about 1.8, RebM/stevioside may be, for example, 0.5 to 50, 1.0 to 45, 1.5 to 40, 2.0 to 35, 2.5 to 30, 3.0 to 25, 3.5 to 20, 4.0 to 15, 4.5 to 10, 4.7 to 8, or 4.8 to 6,, and particularly about 5, (RebD + RebM)/RebA may be, for example, 0.075 to 7.5, 0.150 to 6.75, 0.225 to 6.0, 0.30 to 5.25, 0.375 to 4.5, 0.450 to 3.75, 0.525 to 3.0, 0.60 to 2.25, 0.675 to 1.5, 0.70 to 1.0, or 0.725 to 0.8, and particularly about 0.75, (RebD + RebM)/RebI may be, for example, 0.25 to 25.0, 0.50 to 22.5, 0.75 to 20.0, 1.0 to 17.5, 1.25 to 15.0, 1.50 to 12.5, 1.75 to 10.0, 2.0 to 7.5, 2.1 to 5.0, 2.2 to 4.0, or 2.3 to 3.00, and particularly about 2.5, (RebD + RebM)/stevioside may be, for example, 0.75 to 75.0, 1.50 to 67.5, 2.25 to 60.0, 3.0 to 52.5, 3.75 to 45.0, 4.5 to 37.5, 5.25 to 30.0, 6.0 to 22.5, 6.75 to 15.0, 7.0 to 10.0, or 7.25 to 8.0, and particularly about 7.5, RebE/RebA may be, for example, 0.005 to 0.50, 0.010 to 0.45, 0.015 to 0.40, 0.020 to 0.35, 0.025 to 0.30, 0.030 to 0.25, 0.035 to 0.20, 0.040 to 0.15, 0.045 to 0.10, 0.047 to 0.08, or 0.048 to 0.06, and particularly about 0.05, RebE/RebI may be, for example, 0.025 to 2.50, 0.050 to 2.25, 0.075 to 2.0, 0.10 to 1.75, 0.125 to 1.50, 0.150 to 1.25, 0.175 to 1.0, 0.20 to 0.75, 0.21 to 0.50, 0.22 to 0.40, or 0.23 to 0.30, and particularly about 0.25, and RebE/stevioside may be, for example, 0.05 to 5.0, 0.10 to 4.5, 0.15 to 4.0, 0.20 to 3.5, 0.25 to 3.0, 0.30 to 2.5, 0.35 to 2.0, 0.40 to 1.5, 0.45 to 1.0, 0.47 to 0.8, or 0.48 to 0.6, and particularly about 0.5.

### 4. Extract of transformant

In another aspect, the present invention provides an extract of the above-described transformant (hereinafter, sometimes referred to as the "extract of the present invention"). In a case where the transformant of the present invention has an appropriate substrate or in a case where an appropriate substrate is added from the outside, the content of steviol glycosides is higher than that of the wild type, and thus an extract thereof may contain steviol glycosides, particularly a steviol glycoside in which a hexose is added to position 2 of the monomeric glucose residue at position 13 or position 19 of the steviol glycoside, at a high concentration.

In the extract of the transformant of the present invention, even when the transformant is a cell derived from stevia, the ratio of the steviol glycoside in which a hexose is added to position 2 of the monomeric glucose residue at position 13 or position 19 of the steviol glycoside in the total steviol glycosides is higher than in the extract of wild-type stevia. In the transformant of the present invention, the introduced protein of the present invention is expressed and a hexose is added to position 2 of the monomeric glucose residue at position 13 or position 19 of the steviol glycoside. Thus, when all steviol glycosides are collectively considered, the proportion of the steviol glycoside in which a hexose is added to position 2 of the monomeric glucose residue at position 13 or position 19 of the steviol glycoside is naturally higher than in a cell in which such a protein is not expressed (e.g., a wild-type stevia cell). Such change in the proportion affects characteristics of the extract, for example, the sensory characteristics such as sweetness. Examples of the ratio among steviol glycosides contained in the extract of the present invention are the same as those described above for the steviol glycoside composition of the present invention.

The extract of the transformant of the present invention can be obtained, for example, by disrupting the transformant using glass beads, a homogenizer, a sonicator, or the like, centrifuging the disrupted product, and recovering the supernatant. In a case where the transformant secretes steviol glycosides outside the body, the culture supernatant of the transformant is also included in the extract of the present invention. Further, an additional extraction process may be performed according to the method for extracting steviol glycosides described above.

The extract of the transformant of the present invention can be used, for example, for production of foods, medicaments, industrial raw materials, or the like according to a common method.

In another aspect, the present invention provides a composition including the steviol glycoside composition of the present invention, the transformant, or the extract thereof (hereinafter, sometimes referred to as the "composition of the present invention"). The composition of the present invention may be in any shape, for example, a solid shape (for example, a powder shape or a granular shape), a paste shape, a gel shape, or a liquid shape. The composition of the present invention can have various forms. For example, the composition of the present invention can have forms of foods, food additives, medicaments, quasi-drugs, cosmetics, and the like, or can be used as a raw material for producing these products.

In the present invention, the "food" is a general term for edible products, and includes food defined in each country or region. The food in the present invention include, for example, general foods (including functional foods, nutritional supplements, health supplements, and health foods such as natural food), health-promoting foods (foods for specified health use, foods with functional indicators, foods with nutrient function claims, etc.), and foods for special dietary uses (foods for specified health use, food for the sick, expectant and nursing mothers, lactating woman milk powder, infant formula, foods for dysphagic persons, etc.), in Japan. The food in the present invention also includes infant foods, infant formula, geriatric foods, care foods, medical foods, and the like.

The term "nutritional supplement" refers to a food that is enriched with a certain nutritional component. The term "health food" refers to a food considered healthy or good for health, and includes a nutritional supplement, a natural food, a diet food, and the like. The term "functional food" refers to a food for supplying a nutritional component that performs the regulatory function of the body. The term "infant food" refers to a food for feeding to children up to about 6 years of age. The term "geriatric food" refers to a food that has been treated in such a manner that it is easy to digest and absorb as compared with an untreated food. The term "infant formula" refers to a food for feeding to children up to about 1 year of age. The term "formula for premature infants" refers to a formula to be given to premature infants until they are about 6 months old.

The form of the food is not particularly limited and may be in various forms. Examples of such a form include a beverage, a supplement, and a non-beverage food. The beverage may be either an alcoholic beverage or a non-alcoholic beverage.

Examples of the non-alcoholic beverage include a non-alcoholic beer, a malt beverage, a lactic acid bacterium beverage, a fermented beverage, a cocoa beverage, a sports drink, a nutritional supplement drink, a tea-based beverage (e.g., a tea beverage such as green tea, oolong tea, green tea with roasted rice, or black tea, or a tea beverage produced from plants other than tea plant, such as barley tea, adlay tea, sesame barley tea, buckwheat tea, cherry blossom tea, hydrangea tea, or herbal tea), a soft drink, a coffee beverage (packaged coffee, liquid coffee, or the like), a carbonated beverage (e.g., a cola flavored beverage, a transparent carbonated beverage, a ginger ale, a fruit juice-based carbonated beverage, a milk-containing carbonated beverage or a sugarless carbonated beverage), a functional beverage (e.g., a sports drink, an energy drink, a health support drink, or a pouch jelly drink), a fruit-vegetable beverage (e.g., a 100% fruit juice beverage, a fruit-containing beverage, a low fruit juice-containing soft drink, a berry-containing fruit beverage, or a pulp beverage), a milky beverage (e.g., cow milk, a drink yogurt, a lactic acid bacterium beverage, or a milk-containing soft drink), a soymilk beverage (e.g., soy milk or a soybean beverage), or flavored water.

The alcoholic beverage refers to a beverage containing an alcohol raw material, and may be a white liquor-based beverage. Examples of the alcohol raw material include brewed liquor, distilled liquor, and mixed liquor. Examples of the brewed liquor include wine and beer. Examples of the distilled liquor include spirits (e.g., gin, vodka, rum, tequila, new spirits (drink in which less than 10% of unblended whiskies are mixed), and alcohol as raw material), liqueurs, whiskeys (e.g., whiskey and brandy), and white liquor. Here, the alcoholic beverage only needs to contain a detectable amount of alcohol, and contains, for example, 1 vol.% or more, 2 vol.% or more, 3 vol.% or more, 4 vol.% or more, or 5 vol.% or more of alcohol.

The beverage includes those having various calories. Preferred beverages are low calorie beverages (less than 20 kcal/100 mL) or non-caloric beverages (less than 5 kcal/100 mL).

Examples of the non-beverage foods include, but are not limited to, noodles (buckwheat, udon, Chinese noodles, instant noodles, and the like), tofu, confectioneries (candies, gums, chocolate, snacks, biscuits, cookies, gummies, cakes, wafers, confectionery breads, chocolate, Japanese confectioneries, and the like), breads, fishery or livestock processed foods (fish paste, hams, sausages, and the like), dairy products (fermented milk, butter, cheese, yogurt, and the like), fats and oils and fat processed foods (salad oil, tempura oil, margarine, mayonnaise, shortening, whipped cream, dressing, fat spread, and the like), seasonings (sauce, baste, and the like), cooked or semi-cooked products (champuru, and the like), boil-in-the-bag foods (curries, stews, bowl dishes, rice gruel, mixed porridge, and the like), frozen desserts (ice creams, sherbets, shaved ice, and the like), powdered foods (powdered drinks, powdered soups, and the like), and enteral liquid foods.

Preferred examples of the food in the present invention include beverages and supplements.

The beverage may be prepared as a packaged beverage in a state of being heat-sterilized and packaged in a container. The container is not particularly limited, and examples thereof include a PET bottle, an aluminum can, a steel can, a paper pack, a chilled cup, and a bottle. In a case of performing heat sterilization, the method therefor is not particularly limited, and the heat sterilization can be performed using, for example, a usual method, such as UHT sterilization and retort sterilization. A temperature of the heat sterilization process is not particularly limited and is, for example, from 65 to 130°C and preferably from 85 to 120°C for 10 to 40 minutes. However, if a sterilization value equivalent to that in the above-described conditions is obtained, a sterilization at an appropriate temperature for several seconds, for example, from 5 to 30 seconds, is also acceptable.

The food additive is a product to be added to a food raw material when a food is produced, and may be in a form of, for example, powder, granule, liquid, paste, or the like.

The medicaments include those in various dosage forms. Examples of dosage forms suitable for oral administration include, but are not limited to, powders, granules, tablets, capsules, solutions, suspensions, emulsions, gels, chewable tablets, and syrups, and examples of dosage forms suitable for parenteral administration include injections (e.g., solution injections, suspension injections, emulsion injections, injections prepared before use, and the like), ointments, creams, lotions, poultices, patches, eye drops, nose drops, inhalants, and sprays.

Examples of the quasi-drug include, but are not limited to, dentifrices, mouth fresheners, antiperspirants, medicated cosmetics, hair colors, and sanitary napkins.

Examples of the cosmetics include, but are not limited to, facial cleansers, makeup removers, lotions, serums, packs, and sunscreens.

The composition of the present invention may include a combination of steviol glycosides in the same ratio as the steviol glycoside composition, the transformant, or the extract thereof of the present invention.

The composition of the present invention may also include a component (additional component) other than the steviol glycoside composition, the transformant, or the extract thereof of the present invention. Such a component may vary depending on the form, application, usage, and the like of the composition of the present invention, and non-limiting examples thereof include sweeteners, excipients, binders, disintegrating agents, coating agents, lubricants, coloring agents, flavoring agents, stabilizers, absorption enhancers, pH adjusters, preservatives, antioxidants, perfumes, vitamins, trace metal components, and sour taste components.

Examples of the sweetener include natural sweeteners, sugar alcohols, artificial sweeteners, high-intensity sweeteners, and low-calorie sweeteners. More specific examples thereof include glucose, D-fructose, galactose, mannose, ribose, xylose, maltose, sucrose, lactose, arabinose, rare sugars (e.g., D-tagatose, D-sorbose, D-allulose (D-psicose), L-fructose, L-allulose (L-psicose), L-tagatose, L-sorbose, altrose, D-allose, talose, idose, gulose, and the like), peptide-based sweeteners (e.g., aspartame, neotame, alitame, and the like), sucrose derivative (e.g., sucralose), synthetic sweeteners (e.g., acesulfame K, saccharin, advantem, cyclamate, dulcin, and the like), sugar alcohols (e.g., erythritol, xylitol, sorbitol, maltitol, mannitol, and the like), monellin, curculin, brazein, thaumatin, taumarin, mabinlin. brazein, pentadin, hernandulcin, 4β-hydroxyhernandulcin, miraculin, glycyrrhizin, phyllodulcin, steviol glycosides not contained in the steviol glycoside composition, transformant, or extract thereof of the present invention, Siraitia grosvenorii (Lo Han Guo) extract, mogrosides (e.g., MogV, MogIA1, MogIE1, MogIIA, MogIIA1, MogIIB, MogIIE, MogIII, MogIIIA1, MogIIIE, MobIV, MogIVA, Siamenoside I, and the like) Glycyrrhiza glabra extract, Rubus suavissimus S. Lee extract, Hydrangea macrophylla var. thunbergii (Hydrangea Tea) extract, Sclerochiton ilicifolius extract, Thaumataococcus danielli Benth (Sweetbread arrowroot) extract, Dioscoreophyllum volkensii (serendiptiberry) extract, Curculigo latifolia (Curculigo) extract, Richadella dulcifica (miracle fruit) extract. Pentadiplandra brazzeana extract, Capparis masaikai (Mabinlang) extract, Lippia dulcis (Aztec sweet herb) extract, neohesperidin dihydrochalcone, palatinit, high-fructose corn syrup, fructose-glucose corn syrup, glucose-fructose corn syrup, oligosaccharides, honey, sugarcane juice (black molasses), sugars (white sugar, Sanonto Sugar, brown sugar, Wasambon, and the like), maple syrup, molasses, and starch syrup.

In at least certain exemplary embodiments of the compositions of the present invention, the sweetener as an additional component includes, for example, a sugar alcohol such as erythritol, sorbitol, mannitol, xylitol, lactitol, isomalt, or maltitol. In another embodiment, the sweetener as an additional component includes D-psicose, D-tagatose, a combination of erythritol and D-psicose, or a combination of erythritol, D-tagatose, and D-psicose.

In a case where the composition of the present invention is used as a food, the food may contain a food additive as another component. Examples of the food additive include excipients (e.g., wheat starch, corn starch, cellulose, lactose, sucrose, mannitol, sorbitol, xylitol, pregelatinized starch, casein, magnesium aluminosilicate, calcium silicate, and the like), binders (e.g., pregelatinized starch, hydroxypropylmethylcellulose, polyvinylpyrrolidone, and the like), disintegrating agents (e.g., cellulose, hydroxypropylcellulose, corn starch, and the like), fluidizers (e.g., light anhydrous silicic acid, and the like), oils and/or fats (e.g., vegetable oils such as soybean oil, sesame oil, olive oil, linseed oil, Perilla oil, rapeseed oil, coconut oil, and corn oil, or oils derived from animals and fish), nutrients (e.g., various minerals, various vitamins, and amino acids), flavors, sweeteners, corrigents, colorants, solvents (e.g., ethanol), salts, pH-adjusting agents, buffers, antioxidants, stabilizers, gelling agents, thickeners, lubricants, encapsulating agents, suspending agents, coating agents, antiseptics, and emulsifiers. One of the food additives may be used alone, or two or more thereof may be contained in combination.

In a case where the composition of the present invention is made into a drug or a quasi-drug, the drug or the quasi-drug may contain a pharmaceutically acceptable additive (for example, a surfactant, a carrier, a diluent, an excipient, and the like) as another component. Pharmaceutically acceptable excipients are well known in the medicine field and are described, for example, in Remington's Pharmaceutical Sciences, 18th Ed., Mack Publishing Co., Easton, PA. (1990), which is incorporated herein by reference in its entirety.

The composition of the present invention can be produced by, for example, mixing the steviol glycoside composition of the present invention, the transformant, or the extract thereof with other components as described above as necessary and processing the mixture into a desired form. For example, in a case where the composition of the present invention is in the form of a beverage, the beverage can be produced by a method including adding and mixing components contained in the beverage to a liquid such as water. Accordingly, the present invention relates to a method for producing a steviol glycoside-containing composition (for example, a food, a sweetener composition, a medicament, or the like), the method including combining the steviol glycoside composition, the transformant, or the extract thereof of the present invention with other components (hereinafter, sometimes referred to as the "method for producing a composition of the present invention"). The method for producing a composition of the present invention may further include producing the steviol glycoside composition, the transformant, or the extract thereof of the present invention. The present invention further provides a steviol glycoside-containing composition (for example, a food product, a sweetener composition, a medicament, or the like) obtained by a method including a process of producing the steviol glycoside composition, the transformant, or the extract thereof of the present invention, and combining the steviol glycoside composition, the transformant, or the extract thereof obtained in the process with other components.

The composition of the present invention can be used in a mode suitable for each form. For example, the food can be taken orally, or can be taken by a parenteral nutrition route such as transnasal or enteral. The food additive can be used by the same route as the food in a state of being contained in the food. The medicament can be administered by an administration route depending on the dosage form, such as, but not limited to, oral, buccal, intraoral, intravenous, intramuscular, subcutaneous, intradermal, topical, rectal, intra-articular, intracapsular, intraarterial, intraportal, intraventricular, transmucosal, transdermal, intranasal, intraperitoneal, intratracheal, intratumoral, intracerebral, intrathecal, intracerebroventricular, intrapulmonary, or intrauterine route. The quasi-drug and the cosmetic can also be used depending on the forms thereof, for example, by application to the skin, gargling, or the like.

### 5. Dead tissue or dead cell of transformant

In another aspect, the present invention provides a dead tissue or dead cell of the above-described transformant (hereinafter sometimes referred to as the "dead tissue or dead cell of the present invention"). The term "dead" means a state in which there is no ability to reproduce, proliferate, reproduce, or grow, and includes a case in which death occurs naturally without artificial manipulation, and a case in which death occurs due to artificial manipulation such as cutting, crushing, heating (including heating via a gas such as air, heating via a liquid such as water, and heating via a vapor such as water vapor), freezing, drying, and lyophilization. The "dead tissue" means a tissue in which all cells contained therein are dead, and also includes a dead transformant (transformant in which all cells contained therein are dead). Specific examples of the dead tissue or dead cell of the present invention include a tissue or cell other than seeds, which is dead, in a case where the transformant is a plant transformant. In certain embodiments, the dead tissue or dead cell of the present invention includes dead embryos, meristematic cells, pollen, leaves, roots, root tips, petals, protoplasts, leaf sections, and calluses, which are dead. In certain preferred embodiments, the dead tissue of the present invention is a dried leaf.

In the dead tissue or dead cell of the present invention, in a case where the original transformant contains steviol glycosides, the protein of the present invention is expressed and a hexose is added to position 2 of the monomeric glucose residue at position 13 or position 19 of the steviol glycoside. Thus, in a case where all steviol glycosides contained in dead tissue or dead cell are collectively considered, the proportion of the steviol glycoside in which a hexose is added to position 2 of the monomeric glucose residue at position 13 or position 19 of the steviol glycoside is naturally higher than in an organism in which the protein of the present invention is not expressed (e.g., a wild-type stevia plant body). Such change in proportion affects the characteristics of the dead tissue or dead cell, for example, sensory characteristics such as sweetness. As for the ratio of steviol glycosides contained in the dead tissue or dead cell of the transformant of the present invention containing steviol glycosides, the matter described in "3. Non-human transformant expressing protein of present invention" described above is applied. The dead tissue or dead cell of the present invention itself can be used as a raw material for foods, medicaments, industrial raw materials, or the like.

The present invention further provides the extract of the dead tissue or dead cell of present invention. The present invention further provides a composition including the dead tissue or dead cell of the present invention or the extract thereof. As for the characteristics, forms, and the like of these extracts and compositions, the matters described in "4. Extract of transformant" described above are applied.

### Examples

Hereinafter, the present invention will be more specifically described by using examples, but the present invention is not limited to these examples.

### [Example 1] Construction of yeast expression system

### (1) Construction of expression vector for yeast

To evaluate an enzyme activity of a steviol glycosyltransferase mutant in a yeast expression system, a desired steviol glycosyltransferase gene was inserted into a pESC vector (Agilent Technologies, Catalog #217455) to construct the following expression vector. Note that UGT91D2 (V155T) represents a mutant of UGT91D2 in which a valine residue at position 155 of UGT91D-like3 is substituted with a threonine residue, and UGT91D2 (Y42F/V155T) represents a mutant of UGT91D2 in which a tyrosine residue at position 42 of UGT91D-like3 is substituted with a phenylalanine residue, and a valine residue at position 155 is substituted by a threonine residue.

**[Table 1]**

| Table 1 Configuration of expression vector | | | | |
|---|---|---|---|---|
| | Name | Marker | Insertion gene | Insertion sequence |
| V1 | pESc-Trp-91D2 | TRP | UGT91D-like3 | SEQ ID NO: 13 |
| V2 | pESc-Trp-91D2 (V155T) | TRP | UGT91D2 (V155T) | SEQ ID NO: 14 |
| V3 | pESc-Trp-91D2 (V155T/Y42F) | TRP | UGT91D2 (Y42F/V155T) | SEQ ID NO: 15 |
| V4 | pESc-URA-85C2 | URA | UGT85C2 | SEQ ID NO: 16 |
| V5 | pESc-URA-74G1-85C2 | URA | UGT74G1 + UGT85C2 | SEQ ID NO: 16, 17 |
| V6 | pESc-His-74G1-76G1 | HIS | UGT74G1 + UGT76G1 | SEQ ID NO: 17, 18 |

### (2) Transformation of yeast

Combinations of vectors shown in Table 2 were introduced into a Saccharomyces cerevisiae YPH499 strain as a host by a lithium acetate method, and the following transformants were obtained using a synthetic glucose agar medium lacking an amino acid corresponding to an auxotrophic marker gene.

**[Table 2]**

| Table 2 Configuration of transformants | | | |
|---|---|---|---|
| | Name | Vector | Introduction gene |
| T1 | 91D2/85C2 | V1 + V4 | UGT91D-like3 + UGT85C2 |
| T2 | 91D2 (V155T)/85C2 | V2 + V4 | UGT91D2 (V155T) + UGT85C2 |
| T3 | 91D2/85C2/74G1 | V1 +V5 | UGT91D-like3 + UGT85C2 + UGT74G1 |
| T4 | 91D2 (V155T)/85C2/74G1 | V2 + V5 | UGT91D2 (V155T) + UGT85C2 + UGT74G1 |
| T5 | 91D2/85C2/74G1/76G1 | V1 +V6 | UGT91D-like3 + UGT85C2 + UGT74G1 + UGT76G1 |
| T6 | 91D2 (V155T)/85C2/74G1/76G1 | V2 + V6 | UGT91D2 (V155T) + UGT85C2 + UGT74G1 + UGT76G1 |
| T7 | 91D2 (Y42F/V155T)/85C2/74G1/ 76G1 | V3 + V6 | UGT91D2 (Y42F/V155T) + UGT85C2 + UGT74G1 + UGT76G1 |

### [Example 2] Synthesis of steviol glycoside

A steviol glycoside was synthesized from steviol using the transformant prepared in Example 1. To be specific, after each transformant (2 to 4 clones for each) was cultured overnight at 30°C in 2 mL of a glucose medium (synthetic glucose medium lacking an amino acid corresponding to an auxotrophic marker gene), collected cells were cultured for 2 nights at 30°C in 10 to 15 mL of galactose medium (synthetic galactose medium lacking an amino acid corresponding to an auxotrophic marker gene) supplemented with 10 µg/mL of steviol. After completion of the culture, the culture solution was separated into a supernatant and cells by centrifugation. The culture supernatant was washed with acetonitrile, adsorbed to a Sep-Pak C18 6 cc Vac Cartridge (Waters Corporation) equilibrated with water, washed with 20% acetonitrile, and then eluted with 80% acetonitrile. The dried product was dissolved in 200 µL of 50% acetonitrile, and an amount of the steviol glycoside generated was analyzed by HPLC or LC-MS/MS.

HPLC conditions are as follows.
Column: Shim-pack FC-ODS (4.6 mm × 150 mm, 3 µm, Shimadzu Corporation)
Flow rate: 0.3 mL/min
Temperature: 40°C
Mobile phase A: 0.1% formic acid, mobile phase B: acetonitrile

The analysis by LC-MS/MS was performed in the MRM mode of LCMS8050 (Shimadzu Corporation) using 1 mL of a sample solution.

The results of transformants T1 (91D2/85C2) and T2 (91D2 (V155T)/85C2) are shown in Table 3. The amount of steviol glycosides generated was analyzed by HPLC. Note that numerical values in the table are relative values when an amount of steviolbioside generated by the transformant 1 is taken as 100% (average value excluding a clone with an extremely small production amount).

**[Table 3]**

| Table 3 Steviolbioside generation potential of transformant | |
|---|---|
| | Steviolbioside generation potential (%) |
| T1 | 100.0 |
| T2 | 98.9 |

The results of transformants T3 (91D2/85C2/74G1) and T4 (91D2 (V155T)/85C2/74G1) are shown in Table 4. The amounts of steviol glycosides generated were analyzed by LC-MS/MS, and concentrations of RebA, RebB, RebC, RebD, RebE, RebF, RebG, RebM, RebN, and stevioside were quantified. Numerical values in the table are average values excluding a clone with an extremely low generation amount, and ppm is on a mass basis. In addition, STV represents stevioside, and numerical values in parentheses each represent a ratio of T4 to T3.

**[Table 4]**

| Table 4 RebE generation potential of transformant | | | |
|---|---|---|---|
| | RebE generation amount (ppm) | STV generation amount (ppm) | RebE/STV (%) |
| T3 | 4.88 | 30.3 | 16.1 (100) |
| T4 | 12.8 | 24.6 | 52.0 (323) |

The results of transformants T5 (91D2/85C2/74G1/76G1) and T6 (91D2(V155T)/85C2/74G1/76G1) are shown in Table 5. The amounts of steviol glycosides generated were analyzed by LC-MS/MS, and concentrations of RebA, RebB, RebC, RebD, RebE, RebF, RebG, RebI, RebJ, RebM, RebN, and stevioside were quantified. Numerical values in the table are average values excluding a clone with an extremely low generation amount, and ppm is on a mass basis. STV represents the amount of stevioside generated, and TSG (total steviol glycosides) represents the total generation amount of the steviol glycosides quantified.

**[Table 5]**

| Table 5 Steviol glycoside generation potential of transformant | | | |
|---|---|---|---|
| | T5 | T6 | T6/T5 (%) |
| RebA (ppm) | 45.2 | 25.2 | 55.8 |
| RebB (ppm) | 8.85 | 6.48 | 73.2 |
| RebD (ppm) | 2.46 | 6.19 | 251.6 |
| RebE (ppm) | 0.63 | 1.35 | 214.3 |
| RebG (ppm) | 7.91 | 8.75 | 110.6 |
| RebM (ppm) | 6.64 | 12.8 | 192.8 |
| STV (ppm) | 6.13 | 2.53 | 41.2 |
| RebD + RebM (ppm) | 9.1 | 19.0 | 208.8 |
| TSG (ppm) | 77.8 | 63.2 | 81.3 |
| RebD/RebA | 0.05 | 0.25 | 451.3 |
| RebD/STV | 0.40 | 2.45 | 609.7 |
| RebM/RebA | 0.15 | 0.51 | 345.8 |
| RebM/STV | 1.08 | 5.06 | 467.1 |
| RebE/RebA | 0.01 | 0.05 | 374.5 |
| RebE/STV | 0.10 | 0.53 | 505.9 |
| (RebD + RebM)/RebA | 0.20 | 0.75 | 384.4 |
| (RebD + RebM)/STV | 1.48 | 7.51 | 519.2 |

Table 6 shows results of transformants T5 (91D2/85C2/74G1/76G1), T6 (91D2 (V155T)/85C2/74G1/76G1), and T7 (91D2 (Y43F/V155T)/85C2/74G1/76G1). The amounts of steviol glycosides generated were analyzed by LC-MS/MS, and concentrations of RebA, RebB, RebC, RebD, RebE, RebF, RebI, RebJ, RebM, RebN, and stevioside were quantified. The numerical values in the table are the average values of 4 clones (the numerical range in parentheses is from the minimum value to the maximum value), and ppm is on a mass basis. STV represents the amount of stevioside generated, and TSG (total steviol glycosides) represents the total generation amount of the steviol glycosides quantified. Note that values for RebC, RebF, RebJ, and RebN were not shown because the average values were less than 0.001 ppm.

**[Table 6]**

| Table 6 Steviol glycoside generation potential of transformant | | | | | | |
|---|---|---|---|---|---|---|
| | T5 | T6 | T7 | T6/T5 (%) | T7/T5 (%) | T7/T6 (%) |
| RebA (ppm) | 28.9 (24.1-32.9) | 28.0 (26.2-30.7) | 25.2 (23.6-26.6) | 96.7 | 87.2 | 90.2 |
| RebB (ppm) | 2.43 (2.08-2.86) | 2.62 (2.45-2.74)) | 2.27 (2.05-2.48) | 107.9 | 93.6 | 86.8 |
| RebD (ppm) | 1.48 (1.15-1.67) | 3.67 (3.05-4.35) | 3.90 (3.41-5.08) | 248.9 | 264.0 | 106.0 |
| RebE (ppm) | 0.58 (0.53-0.62) | 1.30 (1.21-1.46) | 1.19 (1.03-1.36) | 223.1 | 203.8 | 91.4 |
| RebI (ppm) | 4.41 (3.41-5.36) | 5.40 (4.81-6.42) | 4.52 (4.17-5.00) | 122.4 | 102.5 | 83.7 |
| RebM (ppm) | 3.20 (2.82-3.77) | 8.62 (7.98-9.48) | 9.16 (8.32-10.47) | 269.3 | 286.0 | 106.2 |
| STV (ppm) | 2.62 (2.03-3.04) | 2.27 (2.20-2.41) | 2.23 (2.01-2.44) | 86.7 | 85.3 | 98.4 |
| RebD + RebM (ppm) | 4.68 (4.28-5.45) | 12.3 (11.0-12.9) | 13.1 (12.0-15.6) | 262.8 | 279.0 | 106.2 |
| TSG (ppm) | 43.6 (37.4-50.2) | 51.9 (48.4-56.3) | 48.5 (44.9-51.9) | 118.8 | 111.1 | 93.5 |
| RebD/RebA | 0.05 (0.04-0.07) | 0.13 (0.10-0.17) | 0.15 (0.13-0.22) | 257.3 | 309.5 | 107.8 |
| RebD/RebI | 0.33 (0.21-0.49) | 0.68 (0.47-0.90) | 0.86 (0.68-1.22) | 203.3 | 257.6 | 126.7 |
| RebD/STV | 0.56 (0.38-0.82) | 1.62 (1.26-1.98) | 1.74 (1.40-2.53) | 287.2 | 309.5 | 107.8 |
| RebM/RebA | 0.11 (0.09-0.16) | 0.31 (0.26-0.36) | 0.36 (0.31-0.44) | 278.4 | 327.8 | 117.8 |
| RebM/RebI | 0.73 (0.53-1.10) | 1.60 (1.24-1.97) | 2.03 (1.66-2.51) | 220.0 | 179.2 | 126.9 |
| RebM/STV | 1.22 (0.93-1.85) | 3.80 (3.31-4.32) | 4.10 (3.41-5.22) | 310.6 | 335.3 | 107.9 |
| RebE/RebA | 0.020 (0.016-0.024) | 0.046 (0.039-0.050) | 0.047 (0.039-0.050) | 230.6 | 233.6 | 101.3 |
| RebE/RebI | 0.13 (0.10-0.18) | 0.24 (0.19-0.30) | 0.26 (0.21-0.33) | 182.2 | 198.9 | 109.2 |
| RebE/STV | 0.22 (0.17-0.31) | 0.57 (0.50-0.66) | 0.53 (0.42-0.68) | 257.3 | 239.0 | 92.9 |
| (RebD + RebM)/RebA | 0.16 (0.13-0.23) | 0.44 (0.36-0.49) | 0.52 (0.45-0.66) | 271.7 | 319.9 | 117.7 |
| (RebD + RebM)/RebI | 1.06 (0.80-1.60) | 2.28 (1.72-2.69) | 2.89 (2.39-3.73) | 214.7 | 272.4 | 126.8 |
| (RebD + RebM)/STV | 1.79 (1.41-2.68) | 5.42 (4.57-5.89) | 5.85 (4.91-7.75) | 303.2 | 327.2 | 107.9 |

The sequences referred to in the present specification are shown below.

**[Table 7]**

| |
|---|
| SEQ ID NO: 1 (UGT91D2 (V155T), CDS) |
| ATGTACAACGTTACTTATCATCAAAATTCAAAAGCAATGGCTACCAGTGACTCCATAGTTGAC GACCGTAAGCAGCTTCATGTTGCGACGTTCCCATGGCTTGCTTTCGGTCACATCCTCCCTTACC TTCAGCTTTCGAAATTGATAGCTGAAAAGGGTCACAAAGTCTCGTTTCTTTCTACCACCAGAAA CATTCAACGTCTCTCTTCTCATATCTCGCCACTCATAAATGTTGTTCAACTCACACTTCCACGTG TCCAAGAGCTGCCGGAGGATGCAGAGGCGACCACTGACGTCCACCCTGAAGATATTCCATATC TCAAGAAGGCTTCTGATGGTCTTCAACCGGAGGTCACCCGGTTTCTAGAACAACACTCTCCGG ACTGGATTATTTATGATTATACTCACTACTGGTTGCCATCCATCGCGGCTAGCCTCGGTATCTC ACGAGCCCACTTCTCCACCACCACTCCATGGGCCATTGCTTATATGGGACCCTCAGCTGACGCC ATGATAAATGGTTCAGATGGTCGAACCACGGTTGAGGATCTCACGACACCGCCCAAGTGGTTT CCCTTTCCGACCAAAGTATGCTGGCGGAAGCATGATCTTGCCCGACTGGTGCCTTACAAAGCTC CGGGGATATCTGATGGATACCGTATGGGGCTGGTTCTTAAGGGATCTGATTGTTTGCTTTCCAA ATGTTACCATGAGTTTGGAACTCAATGGCTACCTCTTTTGGAGACACTACACCAAGTACCGGTG |
| GTTCCGGTGGGATTACTGCCACCGGAAATACCCGGAGACGAGAAAGATGAAACATGGGTGTC AATCAAGAAATGGCTCGATGGTAAACAAAAAGGCAGTGTGGTGTACGTTGCATTAGGAAGCG AGGTTTTGGTGAGCCAAACCGAGGTTGTTGAGTTAGCATTGGGTCTCGAGCTTTCTGGGTTGCC ATTTGTTTGGGCTTATAGAAAACCAAAAGGTCCCGCGAAGTCAGACTCGGTGGAGTTGCCAGA CGGGTTCGTGGAACGAACTCGTGACCGTGGGTTGGTCTGGACGAGTTGGGCACCTCAGTTACG AATACTGAGCCATGAGTCGGTTTGTGGTTTCTTGACTCATTGTGGTTCTGGATCAATTGTGGAA GGGCTAATGTTTGGTCACCCTCTAATCATGCTACCGATTTTTGGGGACCAACCTCTGAATGCTC GATTACTGGAGGACAAACAGGTGGGAATCGAGATACCAAGAAATGAGGAAGATGGTTGCTTG ACCAAGGAGTCGGTTGCTAGATCACTGAGGTCCGTTGTTGTGGAAAAAGAAGGGGAGATCTAC AAGGCGAACGCGAGGGAGCTGAGTAAAATCTATAACGACACTAAGGTTGAAAAAGAATATGT AAGCCAATTCGTAGACTATTTGGAAAAGAATGCGCGTGCGGTTGCCATCGATCATGAGAGTTA A |
| SEQ ID NO: 2 (UGT91D2 (V155T), amino acid sequence) |
| MYNVTYHQNSKAMATSDSIVDDRKQLHVATFPWLAFGHILPYLQLSKLIAEKGHKVSFLSTTRNIQ RLSSHISPLINVVQLTLPRVQELPEDAEATTDVHPEDIPYLKKASDGLQPEVTRFLEQHSPDWIIYDY THYWLPSIAASLGISRAHFSTTTPWAIAYMGPSADAMINGSDGRTTVEDLTTPPKWFPFPTKVCWR KHDLARLVPYKAPGISDGYRMGLVLKGSDCLLSKCYHEFGTQWLPLLETLHQVPVVPVGLLPPEIP GDEKDETWVSIKKWLDGKQKGSVVYVALGSEVLVSQTEVVELALGLELSGLPFVWAYRKPKGPA KSDSVELPDGFVERTRDRGLVWTSWAPQLRILSHESVCGFLTHCGSGSIVEGLMFGHPLIMLPIFGD QPLNARLLEDKQVGIEIPRNEEDGCLTKESVARSLRSVVVEKEGEIYKANARELSKIYNDTKVEKEY VSQFVDYLEKNARAVAIDHES |

**[Table 8]**

| |
|---|
| SEQ ID NO: 3 (UGT91D2 (Y42F/V155T), CDS) |
| ATGTACAACGTTACTTATCATCAAAATTCAAAAGCAATGGCTACCAGTGACTCCATAGTTGAC GACCGTAAGCAGCTTCATGTTGCGACGTTCCCATGGCTTGCTTTCGGTCACATCCTCCCTTTCCT TCAGCTTTCGAAATTGATAGCTGAAAAGGGTCACAAAGTCTCGTTTCTTTCTACCACCAGAAAC ATTCAACGTCTCTCTTCTCATATCTCGCCACTCATAAATGTTGTTCAACTCACACTTCCACGTGT CCAAGAGCTGCCGGAGGATGCAGAGGCGACCACTGACGTCCACCCTGAAGATATTCCATATCT CAAGAAGGCTTCTGATGGTCTTCAACCGGAGGTCACCCGGTTTCTAGAACAACACTCTCCGGA CTGGATTATTTATGATTATACTCACTACTGGTTGCCATCCATCGCGGCTAGCCTCGGTATCTCA CGAGCCCACTTCTCCACCACCACTCCATGGGCCATTGCTTATATGGGACCCTCAGCTGACGCCA TGATAAATGGTTCAGATGGTCGAACCACGGTTGAGGATCTCACGACACCGCCCAAGTGGTTTC CCTTTCCGACCAAAGTATGCTGGCGGAAGCATGATCTTGCCCGACTGGTGCCTTACAAAGCTCC GGGGATATCTGATGGATACCGTATGGGGCTGGTTCTTAAGGGATCTGATTGTTTGCTTTCCAAA TGTTACCATGAGTTTGGAACTCAATGGCTACCTCTTTTGGAGACACTACACCAAGTACCGGTGG TTCCGGTGGGATTACTGCCACCGGAAATACCCGGAGACGAGAAAGATGAAACATGGGTGTCA ATCAAGAAATGGCTCGATGGTAAACAAAAAGGCAGTGTGGTGTACGTTGCATTAGGAAGCGA GGTTTTGGTGAGCCAAACCGAGGTTGTTGAGTTAGCATTGGGTCTCGAGCTTTCTGGGTTGCCA |
| TTTGTTTGGGCTTATAGAAAACCAAAAGGTCCCGCGAAGTCAGACTCGGTGGAGTTGCCAGAC GGGTTCGTGGAACGAACTCGTGACCGTGGGTTGGTCTGGACGAGTTGGGCACCTCAGTTACGA ATACTGAGCCATGAGTCGGTTTGTGGTTTCTTGACTCATTGTGGTTCTGGATCAATTGTGGAAG GGCTAATGTTTGGTCACCCTCTAATCATGCTACCGATTTTTGGGGACCAACCTCTGAATGCTCG ATTACTGGAGGACAAACAGGTGGGAATCGAGATACCAAGAAATGAGGAAGATGGTTGCTTGA CCAAGGAGTCGGTTGCTAGATCACTGAGGTCCGTTGTTGTGGAAAAAGAAGGGGAGATCTACA AGGCGAACGCGAGGGAGCTGAGTAAAATCTATAACGACACTAAGGTTGAAAAAGAATATGTA AGCCAATTCGTAGACTATTTGGAAAAGAATGCGCGTGCGGTTGCCATCGATCATGAGAGTTAA |
| SEQ ID NO: 4 (UGT91D2 (Y42F/V155T), amino acid sequence) |
| MYNVTYHQNSKAMATSDSIVDDRKQLHVATFPWLAFGHILPFLQLSKLIAEKGHKVSFLSTTRNIQ RLSSHISPLINVVQLTLPRVQELPEDAEATTDVHPEDIPYLKKASDGLQPEVTRFLEQHSPDWIIYDY THYWLPSIAASLGISRAHFSTTTPWAIAYMGPSADAMINGSDGRTTVEDLTTPPKWFPFPTKVCWR KHDLARLVPYKAPGISDGYRMGLVLKGSDCLLSKCYHEFGTQWLPLLETLHQVPVVPVGLLPPEIP GDEKDETWVSIKKWLDGKQKGSVVYVALGSEVLVSQTEVVELALGLELSGLPFVWAYRKPKGPA KSDSVELPDGFVERTRDRGLVWTSWAPQLRILSHESVCGFLTHCGSGSIVEGLMFGHPLIMLPIFGD QPLNARLLEDKQVGIEIPRNEEDGCLTKESVARSLRSVVVEKEGEIYKANARELSKIYNDTKVEKEY VSQFVDYLEKNARAVAIDHES |

**[Table 9]**

| |
|---|
| SEQ ID NO: 5 (UGT91D-like3, CDS) |
| ATGTACAACGTTACTTATCATCAAAATTCAAAAGCAATGGCTACCAGTGACTCCATAGTTGAC GACCGTAAGCAGCTTCATGTTGCGACGTTCCCATGGCTTGCTTTCGGTCACATCCTCCCTTACC TTCAGCTTTCGAAATTGATAGCTGAAAAGGGTCACAAAGTCTCGTTTCTTTCTACCACCAGAAA CATTCAACGTCTCTCTTCTCATATCTCGCCACTCATAAATGTTGTTCAACTCACACTTCCACGTG TCCAAGAGCTGCCGGAGGATGCAGAGGCGACCACTGACGTCCACCCTGAAGATATTCCATATC TCAAGAAGGCTTCTGATGGTCTTCAACCGGAGGTCACCCGGTTTCTAGAACAACACTCTCCGG ACTGGATTATTTATGATTATACTCACTACTGGTTGCCATCCATCGCGGCTAGCCTCGGTATCTC ACGAGCCCACTTCTCCGTCACCACTCCATGGGCCATTGCTTATATGGGACCCTCAGCTGACGCC ATGATAAATGGTTCAGATGGTCGAACCACGGTTGAGGATCTCACGACACCGCCCAAGTGGTTT CCCTTTCCGACCAAAGTATGCTGGCGGAAGCATGATCTTGCCCGACTGGTGCCTTACAAAGCTC CGGGGATATCTGATGGATACCGTATGGGGCTGGTTCTTAAGGGATCTGATTGTTTGCTTTCCAA ATGTTACCATGAGTTTGGAACTCAATGGCTACCTCTTTTGGAGACACTACACCAAGTACCGGTG GTTCCGGTGGGATTACTGCCACCGGAAATACCCGGAGACGAGAAAGATGAAACATGGGTGTC AATCAAGAAATGGCTCGATGGTAAACAAAAAGGCAGTGTGGTGTACGTTGCATTAGGAAGCG AGGTTTTGGTGAGCCAAACCGAGGTTGTTGAGTTAGCATTGGGTCTCGAGCTTTCTGGGTTGCC ATTTGTTTGGGCTTATAGAAAACCAAAAGGTCCCGCGAAGTCAGACTCGGTGGAGTTGCCAGA CGGGTTCGTGGAACGAACTCGTGACCGTGGGTTGGTCTGGACGAGTTGGGCACCTCAGTTACG AATACTGAGCCATGAGTCGGTTTGTGGTTTCTTGACTCATTGTGGTTCTGGATCAATTGTGGAA GGGCTAATGTTTGGTCACCCTCTAATCATGCTACCGATTTTTGGGGACCAACCTCTGAATGCTC |
| GATTACTGGAGGACAAACAGGTGGGAATCGAGATACCAAGAAATGAGGAAGATGGTTGCTTG ACCAAGGAGTCGGTTGCTAGATCACTGAGGTCCGTTGTTGTGGAAAAAGAAGGGGAGATCTAC AAGGCGAACGCGAGGGAGCTGAGTAAAATCTATAACGACACTAAGGTTGAAAAAGAATATGT AAGCCAATTCGTAGACTATTTGGAAAAGAATGCGCGTGCGGTTGCCATCGATCATGAGAGTTA A |
| SEQ ID NO: 6 (UGT91D-like3, amino acid sequence) |
| MYNVTYHQNSKAMATSDSIVDDRKQLHVATFPWLAFGHILPYLQLSKLIAEKGHKVSFLSTTRNIQ RLSSHISPLINVVQLTLPRVQELPEDAEATTDVHPEDIPYLKKASDGLQPEVTRFLEQHSPDWIIYDY THYWLPSIAASLGISRAHFSVTTPWAIAYMGPSADAMINGSDGRTTVEDLTTPPKWFPFPTKVCWR KHDLARLVPYKAPGISDGYRMGLVLKGSDCLLSKCYHEFGTQWLPLLETLHQVPVVPVGLLPPEIP GDEKDETWVSIKKWLDGKQKGSVVYVALGSEVLVSQTEVVELALGLELSGLPFVWAYRKPKGPA KSDSVELPDGFVERTRDRGLVWTSWAPQLRILSHESVCGFLTHCGSGSIVEGLMFGHPLIMLPIFGD QPLNARLLEDKQVGIEIPRNEEDGCLTKESVARSLRSVVVEKEGEIYKANARELSKIYNDTKVEKEY VSQFVDYLEKNARAVAIDHES |

**[Table 10]**

| |
|---|
| SEQ ID NO: 7 (UGT74G1, CDS) |
| ATGGCGGAACAACAAAAGATCAAGAAATCACCACACGTTCTACTCATCCCATTCCCTTTACAA GGCCATATAAACCCTTTCATCCAGTTTGGCAAACGATTAATCTCCAAAGGTGTCAAAACAACA CTTGTTACCACCATCCACACCTTAAACTCAACCCTAAACCACAGTAACACCACCACCACCTCCA TCGAAATCCAAGCAATTTCCGATGGTTGTGATGAAGGCGGTTTTATGAGTGCAGGAGAATCAT ATTTGGAAACATTCAAACAAGTTGGGTCTAAATCACTAGCTGACTTAATCAAGAAGCTTCAAA GTGAAGGAACCACAATTGATGCAATCATTTATGATTCTATGACTGAATGGGTTTTAGATGTTGC AATTGAGTTTGGAATCGATGGTGGTTCGTTTTTCACTCAAGCTTGTGTTGTAAACAGCTTATAT TATCATGTTCATAAGGGTTTGATTTCTTTGCCATTGGGTGAAACTGTTTCGGTTCCTGGATTTCC AGAGCTTCAACGGTGGGAGACACCGTTAATTTTGCAGAATCATGAGCAAATACAGAGCCCTTG GTCTCAGATGTTGTTTGGTCAGTTTGCTAATATTGATCAAGCACGTTGGGTCTTCACAAATAGT TTTTACAAGCTCGAGGAAGAGGTAATAGAGTGGACGAGAAAGATATGGAACTTGAAGGTAAT CGGGCCAACACTTCCATCCATGTACCTTGACAAACGACTTGATGATGATAAAGATAACGGATT TAATCTCTACAAAGCAAACCATCATGAGTGCATGAACTGGTTAGACGATAAGCCAAAGGAATC AGTTGTTTACGTAGCATTTGGTAGCCTGGTGAAACATGGACCCGAACAAGTGGAAGAAATCAC ACGGGCTTTAATAGATAGTGATGTCAACTTCTTGTGGGTTATCAAACATAAAGAAGAGGGAAA GCTCCCAGAAAATCTTTCGGAAGTAATAAAAACCGGAAAGGGTTTGATTGTAGCATGGTGCAA ACAATTGGATGTGTTAGCACACGAATCAGTAGGATGCTTTGTTACACATTGTGGGTTCAACTCA ACTCTTGAAGCAATAAGTCTTGGAGTCCCCGTTGTTGCAATGCCTCAATTTTCGGATCAAACTA CAAATGCCAAGCTTCTAGATGAAATTTTGGGTGTTGGAGTTAGAGTTAAGGCTGATGAGAATG GGATAGTGAGAAGAGGAAATCTTGCGTCATGTATTAAGATGATTATGGAGGAGGAAAGAGGA GTAATAATCCGAAAGAATGCGGTAAAATGGAAGGATTTGGCTAAAGTAGCCGTTCATGAAGGT GGTAGCTCAGACAATGATATTGTCGAATTTGTAAGTGAGCTAATTAAGGCTTAA |
| SEQ ID NO: 8 (UGT74G1, amino acid sequence) |
| MAEQQKIKKSPHVLLIPFPLQGHINPFIQFGKRLISKGVKTTLVTTIHTLNSTLNHSNTTTTSIEIQAIS DGCDEGGFMSAGESYLETFKQVGSKSLADLIKKLQSEGTTIDAIIYDSMTEWVLDVAIEFGIDGGSF FTQACVVNSLYYHVHKGLISLPLGETVSVPGFPELQRWETPLILQNHEQIQSPWSQMLFGQFANIDQ ARWVFTNSFYKLEEEVIEWTRKIWNLKVIGPTLPSMYLDKRLDDDKDNGFNLYKANHHECMNWL DDKPKESVVYVAFGSLVKHGPEQVEEITRALIDSDVNFLWVIKHKEEGKLPENLSEVIKTGKGLIVA WCKQLDVLAHESVGCFVTHCGFNSTLEAISLGVPVVAMPQFSDQTTNAKLLDEILGVGVRVKADE NGIVRRGNLASCIKMIMEEERGVIIRKNAVKWKDLAKVAVHEGGSSDNDIVEFVSELIKA |

**[Table 11]**

| |
|---|
| SEQ ID NO: 9 (UGT76G1, CDS) |
| ATGGAAAATAAAACGGAGACCACCGTTCGCCGGCGCCGGAGAATAATATTATTCCCGGTACCA TTTCAAGGCCACATTAACCCAATTCTTCAGCTAGCCAATGTGTTGTACTCTAAAGGATTCAGTA TCACCATCTTTCACACCAACTTCAACAAACCCAAAACATCTAATTACCCTCACTTCACTTTCAG ATTCATCCTCGACAACGACCCACAAGACGAACGCATTTCCAATCTACCGACTCATGGTCCGCTC GCTGGTATGCGGATTCCGATTATCAACGAACACGGAGCTGACGAATTACGACGCGAACTGGAA CTGTTGATGTTAGCTTCTGAAGAAGATGAAGAGGTATCGTGTTTAATCACGGATGCTCTTTGGT ACTTCGCGCAATCTGTTGCTGACAGTCTTAACCTCCGACCGCTTGTTTTGATGACAAGCAGCTT GTTTAATTTTCATGCACATGTTTCACTTCCTCAGTTTGATGAGCTTGGTTACCTCGATCCTGATG ACAAAACCCGTTTGGAAGAACAAGCGAGTGGGTTTCCTATGCTAAAAGTGAAAGACATCAAGT CTGCGTATTCGAACTGGCAAATACTCAAAGAGATATTAGGGAAGATGATAAAACAAACAAAA GCATCTTCAGGAGTCATCTGGAACTCATTTAAGGAACTCGAAGAGTCTGAGCTCGAAACTGTT ATCCGTGAGATCCCGGCTCCAAGTTTCTTGATACCACTCCCCAAGCATTTGACAGCCTCTTCCA GCAGCTTACTAGACCACGATCGAACCGTTTTTCAATGGTTAGACCAACAACCGCCAAGTTCGG TACTGTATGTTAGTTTTGGTAGTACTAGTGAAGTGGATGAGAAAGATTTCTTGGAAATAGCTCG TGGGTTGGTTGATAGCAAGCAGTCGTTTTTATGGGTGGTTCGACCTGGGTTTGTCAAGGGTTCG ACGTGGGTCGAACCGTTGCCAGATGGGTTCTTGGGTGAAAGAGGACGTATTGTGAAATGGGTT CCACAGCAAGAAGTGCTAGCTCATGGAGCAATAGGCGCATTCTGGACTCATAGCGGATGGAAC TCTACGTTGGAAAGCGTTTGTGAAGGTGTTCCTATGATTTTCTCGGATTTTGGGCTCGATCAAC CGTTGAATGCTAGATACATGAGTGATGTTTTGAAGGTAGGGGTGTATTTGGAAAATGGGTGGG AAAGAGGAGAGATAGCAAATGCAATAAGAAGAGTTATGGTGGATGAAGAAGGAGAATACATT AGACAGAATGCAAGAGTTTTGAAACAAAAGGCAGATGTTTCTTTGATGAAGGGTGGTTCGTCT TACGAATCATTAGAGTCTCTAGTTTCTTACATTTCATCGTTGTAA |
| SEQ ID NO: 10 (UGT76G1, amino acid sequence) |
| MENKTETTVRRRRRIILFPVPFQGHINPILQLANVLYSKGFSITIFHTNFNKPKTSNYPHFTFRFILDND PQDERISNLPTHGPLAGMRIPIINEHGADELRRELELLMLASEEDEEVSCLITDALWYFAQSVADSLN LRPLVLMTSSLFNFHAHVSLPQFDELGYLDPDDKTRLEEQASGFPMLKVKDIKSAYSNWQILKEILG KMIKQTKASSGVIWNSFKELEESELETVIREIPAPSFLIPLPKHLTASSSSLLDHDRTVFQWLDQQPPS SVLYVSFGSTSEVDEKDFLEIARGLVDSKQSFLWVVRPGFVKGSTWVEPLPDGFLGERGRIVKWVP |
| QQEVLAHGAIGAFWTHSGWNSTLESVCEGVPMIFSDFGLDQPLNARYMSDVLKVGVYLENGWER GEIANAIRRVMVDEEGEYIRQNARVLKQKADVSLMKGGSSYESLESLVSYISSL |

**[Table 12]**

| |
|---|
| SEQ ID NO: 11 (UGT85C2, CDS) |
| ATGGATGCAATGGCTACAACTGAGAAGAAACCACACGTCATCTTCATACCATTTCCAGCACAA AGCCACATTAAAGCCATGCTCAAACTAGCACAACTTCTCCACCACAAAGGACTCCAGATAACC TTCGTCAACACCGACTTCATCCACAACCAGTTTCTTGAATCATCGGGCCCACATTGTTTGGACG GTTCACCGGGTTTCCGGTTCGAAACCATCCCGGATGGTGTTTCTCACAGTCCGGAAGCGAGCAT CCCAATCAGAGAATCACTCTTGAGATCCATTGAAACCAACTTCTTGGATCGTTTCATTGATCTT GTAACCAAACTTCCGGATCCTCCGACTTGTATTATCTCAGATGGGTTCTTGTCGGTTTTCACAA TTGACGCTGCAAAAAAGCTTGGAATTCCGGTCATGATGTATTGGACACTTGCTGCCTGTGGGTT CATGGGTTTTTACCATATTCATTCTCTCATTGAGAAAGGATTTGCACCACTTAAAGATGCAAGT TACTTGACAAATGGGTATTTGGACACCGTCATTGATTGGGTTCCGGGAATGGAAGGCATCCGT CTCAAGGATTTCCCGCTGGACTGGAGCACTGACCTCAATGACAAAGTTTTGATGTTCACTACAG AAGCTCCTCAAAGGTCACACAAGGTTTCACATCATATTTTCCACACGTTCGATGAGTTGGAGCC TAGTATTATAAAAACTTTGTCATTGAGGTATAATCACATTTACACCATCGGCCCACTGCAATTA CTTCTTGATCAAATACCCGAAGAGAAAAAGCAAACTGGAATTACGAGTCTCCATGGATACAGT TTAGTAAAAGAAGAACCAGAGTGTTTCCAGTGGCTTCAGTCTAAAGAACCAAATTCCGTCGTT TATGTAAATTTTGGAAGTACTACAGTAATGTCTTTAGAAGACATGACGGAATTTGGTTGGGGA CTTGCTAATAGCAACCATTATTTCCTTTGGATCATCCGATCAAACTTGGTGATAGGGGAAAATG CAGTTTTGCCCCCTGAACTTGAGGAACATATAAAGAAAAGAGGCTTTATTGCTAGCTGGTGTTC ACAAGAAAAGGTCTTGAAGCACCCTTCGGTTGGAGGGTTCTTGACTCATTGTGGGTGGGGATC GACCATCGAGAGCTTGTCTGCTGGGGTGCCAATGATATGCTGGCCTTATTCGTGGGACCAGCT GACCAACTGTAGGTATATATGCAAAGAATGGGAGGTTGGGCTCGAGATGGGAACCAAAGTGA AACGAGATGAAGTCAAGAGGCTTGTACAAGAGTTGATGGGAGAAGGAGGTCACAAAATGAGG AACAAGGCTAAAGATTGGAAAGAAAAGGCTCGCATTGCAATAGCTCCTAACGGTTCATCTTCT TTGAACATAGACAAAATGGTCAAGGAAATCACCGTGCTAGCAAGAAACTAG |
| SEQ ID NO: 12 (UGT85C2, amino acid sequence) |
| MDAMATTEKKPHVIFIPFPAQSHIKAMLKLAQLLHHKGLQITFVNTDFIHNQFLESSGPHCLDGSPG FRFETIPDGVSHSPEASIPIRESLLRSIETNFLDRFIDLVTKLPDPPTCIISDGFLSVFTIDAAKKLGIPVM MYWTLAACGFMGFYHIHSLIEKGFAPLKDASYLTNGYLDTVIDWVPGMEGIRLKDFPLDWSTDLN DKVLMFTTEAPQRSHKVSHHIFHTFDELEPSIIKTLSLRYNHIYTIGPLQLLLDQIPEEKKQTGITSLH GYSLVKEEPECFQWLQSKEPNSVVYVNFGSTTVMSLEDMTEFGWGLANSNHYFLWIIRSNLVIGEN AVLPPELEEHIKKRGFIASWCSQEKVLKHPSVGGFLTHCGWGSTIESLSAGVPMICWPYSWDQLTN CRYICKEWEVGLEMGTKVKRDEVKRLVQELMGEGGHKMRNKAKDWKEKARIAIAPNGSSSLNID KMVKEITVLARN |

**[Table 13]**

| |
|---|
| SEQ ID NO: 13 (UGT91D-like3, for vector) |
| GCGGCCGCAAAAATGTACAACGTTACTTATCATCAAAATTCAAAAGCAATGGCTACCAGTGAC TCCATAGTTGACGACCGTAAGCAGCTTCATGTTGCGACGTTCCCATGGCTTGCTTTCGGTCACA TCCTCCCTTACCTTCAGCTTTCGAAATTGATAGCTGAAAAGGGTCACAAAGTCTCGTTTCTTTCT ACCACCAGAAACATTCAACGTCTCTCTTCTCATATCTCGCCACTCATAAATGTTGTTCAACTCA CACTTCCACGTGTCCAAGAGCTGCCGGAGGATGCAGAGGCGACCACTGACGTCCACCCTGAAG ATATTCCATATCTCAAGAAGGCTTCTGATGGTCTTCAACCGGAGGTCACCCGGTTTCTAGAACA ACACTCTCCGGACTGGATTATTTATGATTATACTCACTACTGGTTGCCATCCATCGCGGCTAGC CTCGGTATCTCACGAGCCCACTTCTCCGTCACCACTCCATGGGCCATTGCTTATATGGGACCCT CAGCTGACGCCATGATAAATGGTTCAGATGGTCGAACCACGGTTGAGGATCTCACGACACCGC CCAAGTGGTTTCCCTTTCCGACCAAAGTATGCTGGCGGAAGCATGATCTTGCCCGACTGGTGCC TTACAAAGCTCCGGGGATATCTGATGGATACCGTATGGGGCTGGTTCTTAAGGGATCTGATTGT TTGCTTTCCAAATGTTACCATGAGTTTGGAACTCAATGGCTACCTCTTTTGGAGACACTACACC AAGTACCGGTGGTTCCGGTGGGATTACTGCCACCGGAAATACCCGGAGACGAGAAAGATGAA ACATGGGTGTCAATCAAGAAATGGCTCGATGGTAAACAAAAAGGCAGTGTGGTGTACGTTGCA TTAGGAAGCGAGGTTTTGGTGAGCCAAACCGAGGTTGTTGAGTTAGCATTGGGTCTCGAGCTT TCTGGGTTGCCATTTGTTTGGGCTTATAGAAAACCAAAAGGTCCCGCGAAGTCAGACTCGGTG GAGTTGCCAGACGGGTTCGTGGAACGAACTCGTGACCGTGGGTTGGTCTGGACGAGTTGGGCA CCTCAGTTACGAATACTGAGCCATGAGTCGGTTTGTGGTTTCTTGACTCATTGTGGTTCTGGAT CAATTGTGGAAGGGCTAATGTTTGGTCACCCTCTAATCATGCTACCGATTTTTGGGGACCAACC TCTGAATGCTCGATTACTGGAGGACAAACAGGTGGGAATCGAGATACCAAGAAATGAGGAAG ATGGTTGCTTGACCAAGGAGTCGGTTGCTAGATCACTGAGGTCCGTTGTTGTGGAAAAAGAAG GGGAGATCTACAAGGCGAACGCGAGGGAGCTGAGTAAAATCTATAACGACACTAAGGTTGAA AAAGAATATGTAAGCCAATTCGTAGACTATTTGGAAAAGAATGCGCGTGCGGTTGCCATCGAT CATGAGAGTTAATTAATTAA |
| SEQ ID NO: 14 (UGT91D2 (V155T), for vector) |
| GCGGCCGCAAAAATGTACAACGTTACTTATCATCAAAATTCAAAAGCAATGGCTACCAGTGAC TCCATAGTTGACGACCGTAAGCAGCTTCATGTTGCGACGTTCCCATGGCTTGCTTTCGGTCACA TCCTCCCTTACCTTCAGCTTTCGAAATTGATAGCTGAAAAGGGTCACAAAGTCTCGTTTCTTTCT ACCACCAGAAACATTCAACGTCTCTCTTCTCATATCTCGCCACTCATAAATGTTGTTCAACTCA CACTTCCACGTGTCCAAGAGCTGCCGGAGGATGCAGAGGCGACCACTGACGTCCACCCTGAAG ATATTCCATATCTCAAGAAGGCTTCTGATGGTCTTCAACCGGAGGTCACCCGGTTTCTAGAACA ACACTCTCCGGACTGGATTATTTATGATTATACTCACTACTGGTTGCCATCCATCGCGGCTAGC CTCGGTATCTCACGAGCCCACTTCTCCACCACCACTCCATGGGCCATTGCTTATATGGGACCCT CAGCTGACGCCATGATAAATGGTTCAGATGGTCGAACCACGGTTGAGGATCTCACGACACCGC CCAAGTGGTTTCCCTTTCCGACCAAAGTATGCTGGCGGAAGCATGATCTTGCCCGACTGGTGCC TTACAAAGCTCCGGGGATATCTGATGGATACCGTATGGGGCTGGTTCTTAAGGGATCTGATTGT TTGCTTTCCAAATGTTACCATGAGTTTGGAACTCAATGGCTACCTCTTTTGGAGACACTACACC AAGTACCGGTGGTTCCGGTGGGATTACTGCCACCGGAAATACCCGGAGACGAGAAAGATGAA |
| ACATGGGTGTCAATCAAGAAATGGCTCGATGGTAAACAAAAAGGCAGTGTGGTGTACGTTGCA TTAGGAAGCGAGGTTTTGGTGAGCCAAACCGAGGTTGTTGAGTTAGCATTGGGTCTCGAGCTT TCTGGGTTGCCATTTGTTTGGGCTTATAGAAAACCAAAAGGTCCCGCGAAGTCAGACTCGGTG GAGTTGCCAGACGGGTTCGTGGAACGAACTCGTGACCGTGGGTTGGTCTGGACGAGTTGGGCA CCTCAGTTACGAATACTGAGCCATGAGTCGGTTTGTGGTTTCTTGACTCATTGTGGTTCTGGAT CAATTGTGGAAGGGCTAATGTTTGGTCACCCTCTAATCATGCTACCGATTTTTGGGGACCAACC TCTGAATGCTCGATTACTGGAGGACAAACAGGTGGGAATCGAGATACCAAGAAATGAGGAAG ATGGTTGCTTGACCAAGGAGTCGGTTGCTAGATCACTGAGGTCCGTTGTTGTGGAAAAAGAAG GGGAGATCTACAAGGCGAACGCGAGGGAGCTGAGTAAAATCTATAACGACACTAAGGTTGAA AAAGAATATGTAAGCCAATTCGTAGACTATTTGGAAAAGAATGCGCGTGCGGTTGCCATCGAT CATGAGAGTTAATTAATTAA |

**[Table 14]**

| |
|---|
| SEQ ID NO: 15 (UGT91D2 (Y42F/V155T), for vector) |
| GCGGCCGCAAAAATGTACAACGTTACTTATCATCAAAATTCAAAAGCAATGGCTACCAGTGAC TCCATAGTTGACGACCGTAAGCAGCTTCATGTTGCGACGTTCCCATGGCTTGCTTTCGGTCACA TCCTCCCTTTCCTTCAGCTTTCGAAATTGATAGCTGAAAAGGGTCACAAAGTCTCGTTTCTTTCT ACCACCAGAAACATTCAACGTCTCTCTTCTCATATCTCGCCACTCATAAATGTTGTTCAACTCA CACTTCCACGTGTCCAAGAGCTGCCGGAGGATGCAGAGGCGACCACTGACGTCCACCCTGAAG ATATTCCATATCTCAAGAAGGCTTCTGATGGTCTTCAACCGGAGGTCACCCGGTTTCTAGAACA ACACTCTCCGGACTGGATTATTTATGATTATACTCACTACTGGTTGCCATCCATCGCGGCTAGC CTCGGTATCTCACGAGCCCACTTCTCCACCACCACTCCATGGGCCATTGCTTATATGGGACCCT CAGCTGACGCCATGATAAATGGTTCAGATGGTCGAACCACGGTTGAGGATCTCACGACACCGC CCAAGTGGTTTCCCTTTCCGACCAAAGTATGCTGGCGGAAGCATGATCTTGCCCGACTGGTGCC TTACAAAGCTCCGGGGATATCTGATGGATACCGTATGGGGCTGGTTCTTAAGGGATCTGATTGT TTGCTTTCCAAATGTTACCATGAGTTTGGAACTCAATGGCTACCTCTTTTGGAGACACTACACC AAGTACCGGTGGTTCCGGTGGGATTACTGCCACCGGAAATACCCGGAGACGAGAAAGATGAA ACATGGGTGTCAATCAAGAAATGGCTCGATGGTAAACAAAAAGGCAGTGTGGTGTACGTTGCA TTAGGAAGCGAGGTTTTGGTGAGCCAAACCGAGGTTGTTGAGTTAGCATTGGGTCTCGAGCTT TCTGGGTTGCCATTTGTTTGGGCTTATAGAAAACCAAAAGGTCCCGCGAAGTCAGACTCGGTG GAGTTGCCAGACGGGTTCGTGGAACGAACTCGTGACCGTGGGTTGGTCTGGACGAGTTGGGCA CCTCAGTTACGAATACTGAGCCATGAGTCGGTTTGTGGTTTCTTGACTCATTGTGGTTCTGGAT CAATTGTGGAAGGGCTAATGTTTGGTCACCCTCTAATCATGCTACCGATTTTTGGGGACCAACC TCTGAATGCTCGATTACTGGAGGACAAACAGGTGGGAATCGAGATACCAAGAAATGAGGAAG ATGGTTGCTTGACCAAGGAGTCGGTTGCTAGATCACTGAGGTCCGTTGTTGTGGAAAAAGAAG GGGAGATCTACAAGGCGAACGCGAGGGAGCTGAGTAAAATCTATAACGACACTAAGGTTGAA AAAGAATATGTAAGCCAATTCGTAGACTATTTGGAAAAGAATGCGCGTGCGGTTGCCATCGAT CATGAGAGTTAATTAATTAA |
| SEQ ID NO: 16 (UGT85C2, for vector) |
| AGATCTATGGATGCAATGGCTACAACTGAGAAGAAACCACACGTCATCTTCATACCATTTCCA GCACAAAGCCACATTAAAGCCATGCTCAAACTAGCACAACTTCTCCACCACAAAGGACTCCAG ATAACCTTCGTCAACACCGACTTCATCCACAACCAGTTTCTTGAATCATCGGGCCCACATTGTT TGGACGGTTCACCGGGTTTCCGGTTCGAAACCATCCCGGATGGTGTTTCTCACAGTCCGGAAGC GAGCATCCCAATCAGAGAATCACTCTTGAGATCCATTGAAACCAACTTCTTGGATCGTTTCATT GATCTTGTAACCAAACTTCCGGATCCTCCGACTTGTATTATCTCAGATGGGTTCTTGTCGGTTTT CACAATTGACGCTGCAAAAAAGCTTGGAATTCCGGTCATGATGTATTGGACACTTGCTGCCTGT GGGTTCATGGGTTTTTACCATATTCATTCTCTCATTGAGAAAGGATTTGCACCACTTAAAGATG CAAGTTACTTGACAAATGGGTATTTGGACACCGTCATTGATTGGGTTCCGGGAATGGAAGGCA TCCGTCTCAAGGATTTCCCGCTGGACTGGAGCACTGACCTCAATGACAAAGTTTTGATGTTCAC TACAGAAGCTCCTCAAAGGTCACACAAGGTTTCACATCATATTTTCCACACGTTCGATGAGTTG GAGCCTAGTATTATAAAAACTTTGTCATTGAGGTATAATCACATTTACACCATCGGCCCACTGC AATTACTTCTTGATCAAATACCCGAAGAGAAAAAGCAAACTGGAATTACGAGTCTCCATGGAT ACAGTTTAGTAAAAGAAGAACCAGAGTGTTTCCAGTGGCTTCAGTCTAAAGAACCAAATTCCG TCGTTTATGTAAATTTTGGAAGTACTACAGTAATGTCTTTAGAAGACATGACGGAATTTGGTTG GGGACTTGCTAATAGCAACCATTATTTCCTTTGGATCATCCGATCAAACTTGGTGATAGGGGAA AATGCAGTTTTGCCCCCTGAACTTGAGGAACATATAAAGAAAAGAGGCTTTATTGCTAGCTGG TGTTCACAAGAAAAGGTCTTGAAGCACCCTTCGGTTGGAGGGTTCTTGACTCATTGTGGGTGG GGATCGACCATCGAGAGCTTGTCTGCTGGGGTGCCAATGATATGCTGGCCTTATTCGTGGGAC CAGCTGACCAACTGTAGGTATATATGCAAAGAATGGGAGGTTGGGCTCGAGATGGGAACCAA AGTGAAACGAGATGAAGTCAAGAGGCTTGTACAAGAGTTGATGGGAGAAGGAGGTCACAAAA TGAGGAACAAGGCTAAAGATTGGAAAGAAAAGGCTCGCATTGCAATAGCTCCTAACGGTTCAT CTTCTTTGAACATAGACAAAATGGTCAAGGAAATCACCGTGCTAGCAAGAAACTAGTCGAC |

**[Table 15]**

| |
|---|
| SEQ ID NO: 17 (UGT74G1, for vector) |
| GCGGCCGCATGGCGGAACAACAAAAGATCAAGAAATCACCACACGTTCTACTCATCCCATTCC CTTTACAAGGCCATATAAACCCTTTCATCCAGTTTGGCAAACGATTAATCTCCAAAGGTGTCAA AACAACACTTGTTACCACCATCCACACCTTAAACTCAACCCTAAACCACAGTAACACCACCAC CACCTCCATCGAAATCCAAGCAATTTCCGATGGTTGTGATGAAGGCGGTTTTATGAGTGCAGG AGAATCATATTTGGAAACATTCAAACAAGTTGGGTCTAAATCACTAGCTGACTTAATCAAGAA GCTTCAAAGTGAAGGAACCACAATTGATGCAATCATTTATGATTCTATGACTGAATGGGTTTTA GATGTTGCAATTGAGTTTGGAATCGATGGTGGTTCGTTTTTCACTCAAGCTTGTGTTGTAAACA GCTTATATTATCATGTTCATAAGGGTTTGATTTCTTTGCCATTGGGTGAAACTGTTTCGGTTCCT GGATTTCCAGAGCTTCAACGGTGGGAGACACCGTTAATTTTGCAGAATCATGAGCAAATACAG AGCCCTTGGTCTCAGATGTTGTTTGGTCAGTTTGCTAATATTGATCAAGCACGTTGGGTCTTCA CAAATAGTTTTTACAAGCTCGAGGAAGAGGTAATAGAGTGGACGAGAAAGATATGGAACTTG AAGGTAATCGGGCCAACACTTCCATCCATGTACCTTGACAAACGACTTGATGATGATAAAGAT AACGGATTTAATCTCTACAAAGCAAACCATCATGAGTGCATGAACTGGTTAGACGATAAGCCA |
| AAGGAATCAGTTGTTTACGTAGCATTTGGTAGCCTGGTGAAACATGGACCCGAACAAGTGGAA GAAATCACACGGGCTTTAATAGATAGTGATGTCAACTTCTTGTGGGTTATCAAACATAAAGAA GAGGGAAAGCTCCCAGAAAATCTTTCGGAAGTAATAAAAACCGGAAAGGGTTTGATTGTAGC ATGGTGCAAACAATTGGATGTGTTAGCACACGAATCAGTAGGATGCTTTGTTACACATTGTGG GTTCAACTCAACTCTTGAAGCAATAAGTCTTGGAGTCCCCGTTGTTGCAATGCCTCAATTTTCG GATCAAACTACAAATGCCAAGCTTCTAGATGAAATTTTGGGTGTTGGAGTTAGAGTTAAGGCT GATGAGAATGGGATAGTGAGAAGAGGAAATCTTGCGTCATGTATTAAGATGATTATGGAGGA GGAAAGAGGAGTAATAATCCGAAAGAATGCGGTAAAATGGAAGGATTTGGCTAAAGTAGCCG TTCATGAAGGTGGTAGCTCAGACAATGATATTGTCGAATTTGTAAGTGAGCTAATTAAGGCTT AATTAA |
| SEQ ID NO: 18 (UGT76G1, for vector) |
| GGATCCATGGAAAATAAAACGGAGACCACCGTTCGCCGGCGCCGGAGAATAATATTATTCCCG GTACCATTTCAAGGCCACATTAACCCAATTCTTCAGCTAGCCAATGTGTTGTACTCTAAAGGAT TCAGTATCACCATCTTTCACACCAACTTCAACAAACCCAAAACATCTAATTACCCTCACTTCAC TTTCAGATTCATCCTCGACAACGACCCACAAGACGAACGCATTTCCAATCTACCGACTCATGGT CCGCTCGCTGGTATGCGGATTCCGATTATCAACGAACACGGAGCTGACGAATTACGACGCGAA CTGGAACTGTTGATGTTAGCTTCTGAAGAAGATGAAGAGGTATCGTGTTTAATCACGGATGCT CTTTGGTACTTCGCGCAATCTGTTGCTGACAGTCTTAACCTCCGACCGCTTGTTTTGATGACAA GCAGCTTGTTTAATTTTCATGCACATGTTTCACTTCCTCAGTTTGATGAGCTTGGTTACCTCGAT CCTGATGACAAAACCCGTTTGGAAGAACAAGCGAGTGGGTTTCCTATGCTAAAAGTGAAAGAC ATCAAGTCTGCGTATTCGAACTGGCAAATACTCAAAGAGATATTAGGGAAGATGATAAAACAA ACAAAAGCATCTTCAGGAGTCATCTGGAACTCATTTAAGGAACTCGAAGAGTCTGAGCTCGAA ACTGTTATCCGTGAGATCCCGGCTCCAAGTTTCTTGATACCACTCCCCAAGCATTTGACAGCCT CTTCCAGCAGCTTACTAGACCACGATCGAACCGTTTTTCAATGGTTAGACCAACAACCGCCAA GTTCGGTACTGTATGTTAGTTTTGGTAGTACTAGTGAAGTGGATGAGAAAGATTTCTTGGAAAT AGCTCGTGGGTTGGTTGATAGCAAGCAGTCGTTTTTATGGGTGGTTCGACCTGGGTTTGTCAAG GGTTCGACGTGGGTCGAACCGTTGCCAGATGGGTTCTTGGGTGAAAGAGGACGTATTGTGAAA TGGGTTCCACAGCAAGAAGTGCTAGCTCATGGAGCAATAGGCGCATTCTGGACTCATAGCGGA TGGAACTCTACGTTGGAAAGCGTTTGTGAAGGTGTTCCTATGATTTTCTCGGATTTTGGGCTCG ATCAACCGTTGAATGCTAGATACATGAGTGATGTTTTGAAGGTAGGGGTGTATTTGGAAAATG GGTGGGAAAGAGGAGAGATAGCAAATGCAATAAGAAGAGTTATGGTGGATGAAGAAGGAGA ATACATTAGACAGAATGCAAGAGTTTTGAAACAAAAGGCAGATGTTTCTTTGATGAAGGGTGG TTCGTCTTACGAATCATTAGAGTCTCTAGTTTCTTACATTTCATCGTTGTAAGTCGAC |

## Claims

1. A protein selected from:
(a) a protein comprising the amino acid sequence of SEQ ID NO: 2;
(b) a protein comprising an amino acid sequence wherein 1 to 48 amino acid residues other than the amino acid residue at position 155 are deleted, substituted, inserted, and/or added in the amino acid sequence of SEQ ID NO: 2 and having an activity to add hexose at position 2 of a monomeric glucose residue at position 13 or position 19 in a compound represented by Formula (I); and
(c) a protein comprising an amino acid sequence having a sequence identity of 90% or more to the amino acid sequence of SEQ ID NO: 2 but in which an amino acid residue corresponding to the amino acid residue at position 155 is the same and having an activity to add hexose at position 2 in the monomeric glucose residue at position 13 or 19 in the compound represented by Formula (I): 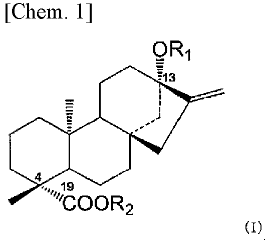
wherein R₁ and R₂ each independently represent H, a C₁ to C₂₀ alkyl group, a C₂ to C₂₀ alkenyl group, a C₂ to C₂₀ alkynyl group, a C₄ to C₂₀ alkyldienyl group, a C₆ to C₁₈ aryl group, a C₆ to C₂₀ alkylaryl group, a C₆ to C₂₀ arylalkyl group, a C₄ to C₂₀ cycloalkyl group, a C₄ to C₂₀ cycloalkenyl group, a (C₃ to C₁₀ cycloalkyl)C₁ to C₁₀ alkyl group, or a sugar residue, with the proviso that R₁ and/or R₂ is a monomeric glucose residue.

2. The protein according to claim 1, wherein the amino acid residue at position 42 in the amino acid sequence of SEQ ID NO: 2 or an amino acid residue corresponding thereto is substituted with a phenylalanine residue.

3. The protein according to claim 1 or 2, wherein the hexose is glucose or rhamnose.

4. The protein according to any one of claims 1 to 3, wherein the compound represented by Formula (I) is steviolmonoside, rubusoside, or stevioside.

5. A polynucleotide encoding the protein according to any one of claims 1 to 4.

6. A vector comprising the polynucleotide according to claim 5.

7. A non-human transformant comprising the polynucleotide according to claim 5.

8. The transformant according to claim 7, comprising UGT85C2, UGT76G1, and/or UGT74G1.

9. An extract of the transformant according to claim 7 or 8.

10. A food, a medicament, or an industrial raw material comprising the extract according to claim 9.

11. A method for producing the protein according to any one of claims 1 to 4, the method comprising culturing the non-human transformant according to claim 7 or 8.

12. A method for producing a steviol glycoside or a steviol glycoside composition, the method comprising culturing the non-human transformant according to claim 7 or 8.

13. A method for producing a steviol glycoside or a steviol glycoside composition, the method comprising reacting the protein according to any one of claims 1 to 4, a UDP-sugar, and the compound represented by Formula (I).

14. The method according to claim 13, wherein the UDP-sugar is UDP-glucose or UDP-rhamnose.

15. The method according to any one of claims 12 to 14, wherein the steviol glycoside composition comprises RebE, steviolbioside, stevioside, and/or dulcoside A.
